# EUROPEAN PATENT APPLICATION

(11) **EP 4 729 471 A2**
(43) Date of publication of application: **22.04.2026**
(21) Application number: 26161365.7
(22) Date of filing: 07.05.2020
(51) Int. Cl.: B82Y 15/00

(54) **NOVEL LIGAND ASSAYS**

(30) Priority: 07.05.2019 NZ 19753245
(62) Divisional of application: 20801845.7
(71) Applicant: InsituGen Limited, Dunedin 9016 (NZ)
(72) Inventor: HEATHER, Alison Kay, 9013 Dunedin (NZ); SOWERBY, Stephen John, 9076 Dunedin (NZ)
(74) Representative: J A Kemp LLP

(57) **Abstract**

The present invention is concerned with the detection of ligands which bind to and activate steroid hormone receptors. Specifically, the present invention provides test kits and assay methods for the selective identification of steroid hormone receptor ligands from a test sample. Importantly, the test kits and assay methods described herein are cell-free, and do not require expensive-to-manufacture nuclear extracts for their performance. Instead, the test kits and assay methods described herein employ single polypeptide polymerases, such as T7 RNA polymerase, linked to a reporter construct. Activity of the enzyme is inhibited, rather than activated, by ligand-bound steroid hormone receptor complexes which only form in the presence of a target ligand. Accordingly, a measured change in a physical property of the reporter construct (e.g. fluorescence output) may be used to determine the presence of a target ligand in a sample under investigation.

## Description

### TECHNICAL FIELD

The invention relates generally to assays, methods and test kits for detection of a ligand in a sample. In particular, the present invention provides assays, methods and test kits to screen a test sample for the presence of a ligand characterized by its ability to form a complex with a steroid hormone receptor to elicit a steroid hormone specific genomic response.

### BACKGROUND OF THE INVENTION

The detection of ligands capable of eliciting a steroid hormone genomic response is important in many areas of biochemistry, molecular biology and medicine. Such ligands include endogenous steroids, exogenous steroids, non-steroidal and synthetic molecules. For example, the determination of total hormone bioactivity in serum or plasma is important for monitoring human health related conditions including aging, perimenopause, menopause, hypoandrogenism, hyperandrogenism, hormone replacement therapy, endocrine cancers including breast and prostate cancers, other hormone related conditions such as osteoporosis and liver toxicity, irregular menstruation, polycystic ovary syndrome, disorders of sexual development and infertility. Conventional detection methods for androgenic/estrogenic and antiandrogenic/antiestrogenic molecules provide no information about hormone biological activity. Hormone biological activity is an important measurement for understanding underlying mechanisms that are driving health conditions so that appropriate treatments/interventions can be implemented.

The detection of hormonal bioactivity in samples is also important for monitoring illicit human and animal performance enhancement, injury cover-up, supplement and food adulteration, growth promoters in dairy industry and environmental pollutants. Measuring hormonal bioactivity provides information about contaminants and/or adulterants that are likely to modulate endocrine pathways in the body, thereby affecting human health.

Ligands that elicit a steroid hormone genomic response first activate steroid hormone receptor proteins by forming a complex with them in the cytoplasm or nucleus of eukaryote cells to form an activated receptor protein. The ligand displaces co-factors on the receptor protein, which then exposes DNA binding motifs. The activated receptor protein dimerises with a second activated receptor protein and translocates to the nucleus if need be to interact with DNA by binding to a specific nucleotide sequence called a response element. In normal biological function, the assemblage of ligand-activated steroid hormone receptor proteins bound to a response element regulates gene expression by enhancing or repressing the initiation of RNA polymerase II mediated transcription. RNA polymerase II is a multi-subunit holoenzyme that assembles to catalyse RNA transcription by polymerising nucleotide triphosphates against a DNA template.

The steroid hormone genomic response is induced by ligands that bind to steroid hormone receptor proteins and receptor-specific response elements for example androgen receptor (AR) and the androgen response element (ARE), estrogen receptor-α, (ER-α), estrogen receptor-β (ER-β) and the estrogen response element (ERE), glucocorticoid receptor (GR) and the glucocorticoid response element (GRE), mineralocorticoid receptor (MR) and the mineralocorticoid response element (MRE), progesterone receptor-A (PR-A), progesterone receptor-B (PR-B) and the progesterone response element (PRE).

However, not all ligands that bind to steroid hormone receptor proteins elicit a steroid hormone genomic response. Some ligands elicit a non-genomic response that is characterised by second messenger signalling, such as G-protein activation. Such non-genomic responses occur within seconds to minutes of ligand binding, and are not a classical steroid hormone response.

A common way to detect the presence of a ligand in a sample is to measure it directly in that sample. However, samples are often complex mixtures of molecules and typically require a complicated process of preparation for analysis. Detecting the presence of a ligand(s) in a sample typically relies on processes such as liquid or gas chromatography to separate the molecular species from a complex mixture into fractions of relatively pure composition and then analyse each fraction with a structure-sensitive method such as mass spectrometry. More than 100 ligands can be tested in any one sample using this approach. Automated purification systems, gas or liquid chromatograms, and mass spectrometers are costly and technically complicated laboratory instruments that must be continually calibrated and operated by trained technicians in order to produce reliable results. Another disadvantage is that some ligands may be rendered biologically inactive by interaction with proteins such as sex hormone binding globulin or serum albumin and this methodology does not distinguish between biologically active and inactive fractions of ligands. Also, the process of ionization can lead to disintegration of some steroid molecules such that they cannot be measured using such methodologies. Additionally, this methodology does not provide information about the total biological activity of a sample from multiple ligands when all known ligands cannot be identified or where ligands may be identified it is not known if the activity would be additive, synergistic, or even competitive. Furthermore, prior knowledge of the molecular structure of the ligand(s) and its associated metabolite(s) due to the biological metabolism of the ligand(s) is required to achieve reliable identification of the presence of ligand(s) in the sample.

Another common way to detect the presence of a steroid hormone ligand in a sample is to use biological assays based on immunological techniques, such as radioimmunoassay and enzyme-linked immunosorbent assay. A limitation of immunological techniques is the requirement for antibody molecules to detect the ligands directly or the ligands bound to sex hormone binding globulin. Immunological assays lack reproducibility due to the high degree of variability in the antibody molecules produced by different manufactures of the assays.

To overcome limitations with detection of ligands in a sample, cell-based steroid hormone assays have been developed in which ligands bind to steroid hormone receptor proteins and elicit a steroid hormone genomic response. In these assays, the presence of a ligand in a sample is detected after the steroid hormone receptor is activated and increases RNA polymerase II transcription of a reporter gene, which is then translated into a protein. Most commonly, the reporter gene encodes a fluorescent protein (such as GFP) or an enzyme that will induce a light or colorimetric reaction in the presence of specific substrate.

However, there are significant limitations associated with these cell-based assays in that they require specialised equipment and expertise to maintain living cell cultures. This increases the cost of cell-based testing and reduces high throughput application of these methods. Additionally, the high level of molecular complexity of a living cell makes testing difficult and reduces both specificity and reproducibility.

To overcome the limitations of detecting a ligand in a sample using cell-based assays, cell-free systems that detect a ligand in a sample where the ligand is capable of eliciting a steroid hormone genomic response have been developed.

However, a limitation of the cell-free assays is the requirement to use multi-subunit holoenzyme polymerases, such as RNA Polymerase II. The recombinant production of RNA polymerase II is extremely difficult to achieve with variable reproducibility, and as a consequence, the holoenzyme is typically made available by using nuclear extracts where all the components exist and come together at the promoter sequence. However, preparing nuclear extracts from eukaryotic cells is expensive to manufacture because of the need for costly cell growth media and the technical expertise required to enrich nuclear materials.

Specifically, the present invention provides test kits, assays and methods involving single polypeptide polymerases, the activity of which is reduced or inhibited, rather than activated, by ligands that bind steroid hormone receptor proteins.

### SUMMARY OF THE INVENTION

The inventions described and claimed herein have many attributes and examples including, but not limited to, those set forth or described or referenced in this Summary of the Invention. It is not intended to be all-inclusive and the inventions described and claimed herein are not limited to or by the features or examples identified in this Summary of the Invention, which is included for purposes of illustration only and not restriction.

In an aspect of the present invention there is provided a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid molecule comprising:
   (a) a polymerase promoter sequence;
   (b) a response element that is capable of being bound by the receptor-ligand complex; and
   (c) a reporter construct
   where the response element (b) is located between the promoter sequence (a) and the reporter construct (c), and (a), (b) and (c) are operably linked; and
(iii) a polymerase,
wherein, the presence of a ligand in the sample is detected by measuring a reduction or inhibition in transcription of the reporter construct caused by binding of the ligand-receptor complex to the response element when the sample is combined with the test kit.

In a further aspect of the present invention there is provided a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid molecule comprising:
   (a) a polymerase promoter sequence;
   (b) a response element that is capable of being bound by the receptor-ligand complex; and
   (c) a reporter construct
   where the response element (b) is located between the promoter sequence (a) and the reporter construct (c), and (a), (b) and (c) are operably linked; and
(iii) a single polypeptide polymerase,
wherein, the presence of a ligand in the sample is detected by measuring a reduction or inhibition in transcription of the reporter construct caused by binding of the ligand-receptor complex to the response element when the sample is combined with the test kit.

In a further aspect of the present invention there is provided a test kit for screening a sample for the presence of a ligand capable of eliciting a steroid hormone genomic response, the test kit comprising:
(i) a steroid hormone receptor that is capable of forming a receptor-ligand complex with a ligand from the sample; and
(ii) a nucleic acid molecule comprising:
   (a) a polymerase promoter sequence;
   (b) a response element that is capable of being bound by the receptor-ligand complex; and
   (c) a reporter construct
   where the response element (b) is located between the promoter sequence (a) and the reporter construct (c), and (a), (b) and (c) are operably linked; and
(iii) a single polypeptide RNA polymerase,
wherein, the presence of a ligand in the sample is detected by measuring a reduction or inhibition in transcription of the reporter construct caused by binding of the ligand-receptor complex to the response element when the sample is combined with the test kit.

In yet a further aspect of the present invention there is provided a test kit for screening a sample for the presence of a ligand capable of eliciting a steroid hormone genomic response, the test kit comprising:
(i) a steroid hormone receptor that is capable of forming a receptor-ligand complex with a ligand from the sample;
(ii) a nucleic acid molecule comprising:
   (a) a T7 RNA polymerase promoter sequence;
   (b) a response element that is capable of being bound by the receptor-ligand complex; and
   (c) a reporter construct
   where the response element (b) is located between the promoter sequence (a) and the reporter construct (c), and (a), (b) and (c) are operably linked; and
(iii) a T7 RNA polymerase,
wherein, the presence of a ligand in the sample is detected by measuring a reduction or inhibition in transcription of the reporter construct caused by binding of the ligand-receptor complex to the response element when the sample is combined with the test kit.

In yet a further aspect of the present invention there is provided a test kit for screening a sample for the presence of a ligand capable of eliciting a steroid hormone genomic response, the test kit comprising:
(i) a steroid hormone receptor that is capable of forming a receptor-ligand complex with a ligand from the sample;
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52;
(iii) a nucleic acid molecule comprising:
   (a) a T7 RNA polymerase promoter sequence defined by SEQ ID NO: 1;
   (b) a response element that is capable of being bound by the receptor-ligand complex; and
   (c) a reporter construct
   where the response element (b) is located between the promoter sequence (a) and the reporter construct (c), and (a), (b) and (c) are operably linked; and
(iv) a T7 RNA polymerase,
wherein, the presence of a ligand in the sample is detected by measuring a reduction or inhibition in transcription of the reporter construct caused by binding of the ligand-receptor complex to the response element when the sample is combined with the test kit.

In yet a further aspect of the present invention there is provided a test kit for screening a sample for the presence of a ligand capable of eliciting a steroid hormone genomic response, the test kit comprising:
(i) a steroid hormone receptor that is capable of forming a receptor-ligand complex with a ligand from the sample;
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52;
(iii) a nucleic acid molecule comprising:
   (a) a T7 RNA polymerase promoter sequence defined by SEQ ID NO: 1;
   (b) a response element that is capable of being bound by the receptor-ligand complex; and
   (c) a reporter construct comprising an RNA aptamer which is capable of binding to a fluorophore
   where the response element (b) is located between the promoter sequence (a) and the reporter construct (c), and (a), (b) and (c) are operably linked; and
(iv) a T7 RNA polymerase,
wherein, the presence of a ligand in the sample is detected by measuring a reduction or inhibition in fluorescence of the reporter construct caused by binding of the ligand-receptor complex to the response element when the sample is combined with the test kit.

In yet a further aspect of the present invention there is provided a test kit for screening a sample for the presence of a ligand capable of eliciting a steroid hormone genomic response, the test kit comprising:
(i) a steroid hormone receptor that is capable of forming a receptor-ligand complex with a ligand from the sample;
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52;
(iii) a nucleic acid molecule comprising:
   (a) a T7 RNA polymerase promoter sequence defined by SEQ ID NO: 1;
   (b) a response element that is capable of being bound by the receptor-ligand complex; and
   (c) a reporter construct comprising an RNA aptamer which is capable of binding to a fluorophore,
   where the response element (b) is located between the promoter sequence (a) and the reporter construct (c), and (a), (b) and (c) are operably linked;
(iv) a fluorophore which is capable of binding to the RNA aptamer; and
(v) a T7 RNA polymerase,
wherein, the presence of a ligand in the sample is detected by measuring a reduction or inhibition in fluorescence of the reporter construct caused by binding of the ligand-receptor complex to the response element when the sample is combined with the test kit.

In yet a further aspect of the present invention there is provided a test kit for screening a sample for the presence of a ligand capable of eliciting a steroid hormone genomic response, the test kit comprising:
(i) a steroid hormone receptor that is capable of forming a receptor-ligand complex with a ligand from the sample;
(ii) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52;
(iii) a nucleic acid molecule comprising:
   (a) a T7 RNA polymerase promoter sequence defined by SEQ ID NO: 1;
   (b) a response element that is capable of being bound by the receptor-ligand complex; and
   (c) a reporter construct comprising a Mango aptamer which is capable of binding to thiazole orange,
   where the response element (b) is located between the promoter sequence (a) and the reporter construct (c), and (a), (b) and (c) are operably linked;
(iv) thiazole orange; and
(v) a T7 RNA polymerase,
wherein, the presence of a ligand in the sample is detected by measuring a reduction or inhibition in fluorescence of the reporter construct caused by binding of the ligand-receptor complex to the response element when the sample is combined with the test kit.

In yet another aspect of the present invention there is provided an assay method for detecting a ligand in a sample which ligand is capable of eliciting a steroid hormone genomic response, the assay method comprising the steps of:
(i) contacting a sample with:
   (a) a steroid hormone receptor that forms a ligand-receptor complex with a ligand from the sample; and
   (b) a nucleic acid molecule comprising:
      (1) a polymerase promoter sequence;
      (2) a response element that is bound by the ligand-receptor complex; and
      (3) a reporter construct
      where the response element (b) is located between the promoter sequence (a) and the reporter construct (c), and (a), (b) and (c) are operably linked;
   (c) a single polypeptide polymerase; and
   (d) nucleoside triphosphates; and
(ii) measuring a reduction or inhibition in transcription of the reporter construct caused by binding of the ligand-receptor complex to the response element,
wherein, a measured reduction or inhibition in transcription of the reporter construct reflects detection of a ligand in the sample.

In yet another aspect of the present invention there is provided an assay method for detecting a ligand in a sample which ligand is capable of eliciting a steroid hormone genomic response, the assay method comprising the steps of:
(i) contacting a sample with:
   (a) a steroid hormone receptor that forms a ligand-receptor complex with a ligand from the sample; and
   (b) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52;
   (c) a nucleic acid molecule comprising:
      (1) a polymerase promoter sequence;
      (2) a response element that is bound by the ligand-receptor complex; and
      (3) a reporter construct
      where the response element (b) is located between the promoter sequence (a) and the reporter construct (c), and (a), (b) and (c) are operably linked;
   (d) a single polypeptide polymerase; and
   (e) nucleoside triphosphates; and
(ii) measuring a reduction or inhibition in transcription of the reporter construct caused by binding of the ligand-receptor complex to the response element,
wherein, a measured reduction or inhibition in transcription of the reporter construct reflects detection of a ligand in the sample.

In yet another aspect of the present invention there is provided an assay method for detecting a ligand in a sample which ligand is capable of eliciting a steroid hormone genomic response, the method comprising the steps of:
(i) contacting a sample with a test kit as described herein; and
(ii) measuring a reduction or inhibition in transcription of the reporter construct caused by binding of the ligand-receptor complex to the response element,
wherein, a measured reduction or inhibition in transcription of the reporter construct reflects detection of a ligand in the sample.

In yet another aspect of the present invention there is provided an assay method for detecting a ligand in a sample which ligand is capable of eliciting a steroid hormone genomic response, the assay method comprising the steps of:
(i) contacting a sample with:
   (a) a steroid hormone receptor that forms a ligand-receptor complex with a ligand from the sample; and
   (b) a nucleic acid molecule comprising:
      (1) a polymerase promoter sequence;
      (2) a response element that is bound by the ligand-receptor complex; and
      (3) a fluorescence based reporter construct
      where the response element (b) is located between the promoter sequence (a) and the reporter construct (c), and (a), (b) and (c) are operably linked;
   (c) a single polypeptide polymerase; and
   (d) nucleoside triphosphates; and
(ii) measuring a reduction or inhibition in fluorescence of the reporter construct caused by binding of the ligand-receptor complex to the response element,
wherein, a measured reduction or inhibition in fluorescence of the reporter construct reflects detection of a ligand in the sample.

In yet another aspect of the present invention there is provided an assay method for detecting a ligand in a sample which ligand is capable of eliciting a steroid hormone genomic response, the assay method comprising the steps of:
(i) contacting a sample with:
   (a) a steroid hormone receptor that forms a ligand-receptor complex with a ligand from the sample; and
   (b) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52;
   (c) a nucleic acid molecule comprising:
      (1) a polymerase promoter sequence;
      (2) a response element that is bound by the ligand-receptor complex; and
      (3) a fluorescence based reporter construct
      where the response element (b) is located between the promoter sequence (a) and the reporter construct (c), and (a), (b) and (c) are operably linked;
   (d) a single polypeptide polymerase; and
   (e) nucleoside triphosphates; and
(ii) measuring a reduction or inhibition in fluorescence of the reporter construct caused by binding of the ligand-receptor complex to the response element,
wherein, a measured reduction or inhibition in fluorescence of the reporter construct reflects detection of a ligand in the sample.

In yet another aspect of the present invention there is provided an assay method for detecting a ligand in a sample which ligand is capable of eliciting a steroid hormone genomic response, the method comprising the steps of:
(i) contacting a sample with a test kit comprising a fluorescence-based reporter construct as described herein; and
(ii) measuring a reduction or inhibition in fluorescence of the reporter construct caused by binding of the ligand-receptor complex to the response element,
wherein, a measured reduction or inhibition in fluorescence of the reporter construct reflects detection of a ligand in the sample.

In a further aspect of the present invention there is provided a method for determining the steroid hormone bioactivity of a sample, the method comprising combining a sample with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the sample.

In a further aspect of the present invention there is provided a method for determining the steroid hormone bioactivity of a biological sample, the method comprising combining a biological sample with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the biological sample.

In a further aspect of the present invention there is provided a method for determining the steroid hormone bioactivity of a clinical specimen, the method comprising combining a sample obtained from the clinical specimen with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the clinical specimen.

In a further aspect of the present invention there is provided a method for determining the steroid hormone bioactivity of a food or a nutritional supplement, the method comprising combining the food or nutritional supplement, or an extract of the food or nutritional supplement, with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the food or nutritional supplement.

In a further aspect of the present invention there is provided a method for determining the steroid hormone bioactivity of a sample derived from an environmental source, the method comprising combining a sample obtained from an environmental source with a test kit as described herein to ascertain if the sample comprises a ligand sufficient to activate a steroid hormone receptor and cause a change in a physical property of the reporter construct, wherein a change in a physical property of the reporter construct provides information about the steroid hormone bioactivity of the environmental sample.

In a further aspect of the present invention there is provided a method for determining the doping status of an athlete, the method comprising combining a sample obtained from an athlete with a test kit as described herein to ascertain if the sample comprises a ligand sufficient to activate a steroid hormone receptor and cause a change in a physical property of the reporter construct, wherein a change in a physical property of the reporter construct provides information about the doping status of the athlete.

In a further aspect of the present invention there is provided a method for determining the steroid hormone bioactivity of a sample, the method comprising performing an assay method as described herein on a sample to ascertain if the sample comprises a ligand sufficient to activate a steroid hormone receptor and cause a change in a physical property of the reporter construct, wherein a change in a physical property of the reporter construct provides information about the steroid hormone bioactivity of the sample.

In a further aspect of the present invention there is provided a method for determining the steroid hormone bioactivity of a biological sample, the method comprising performing an assay method as described herein on a sample to ascertain if the sample comprises a ligand sufficient to activate a steroid hormone receptor and cause a change in a physical property of the reporter construct, wherein a change in a physical property of the reporter construct provides information about the steroid hormone bioactivity of the biological sample.

In a further aspect of the present invention there is provided a method for determining the steroid hormone bioactivity of a food or a nutritional supplement, the method comprising performing an assay method as described herein on the food or nutritional supplement, or an extract from the food or nutritional supplement, to ascertain if the food or nutritional supplement comprises a ligand sufficient to activate a steroid hormone receptor and cause a change in a physical property of the reporter construct, wherein a change in a physical property of the reporter construct provides information about the steroid hormone bioactivity of the food or nutritional supplement.

In a further aspect of the present invention there is provided a method for determining the steroid hormone bioactivity of a clinical specimen, the method comprising performing an assay method as described herein on a clinical specimen to ascertain if the sample comprises a ligand sufficient to activate a steroid hormone receptor and cause a change in a physical property of the reporter construct, wherein a change in a physical property of the reporter construct provides information about the steroid hormone bioactivity of the clinical specimen.

In a further aspect of the present invention there is provided a method for determining the steroid hormone bioactivity of a sample derived from an environmental source, the method comprising performing an assay method as described herein on the environmental sample to ascertain if the sample comprises a ligand sufficient to activate a steroid hormone receptor and cause a change in a physical property of the reporter construct, wherein a change in a physical property of the reporter construct provides information about the steroid hormone bioactivity.

In a further aspect of the present invention there is provided a method for determining the doping status of an athlete, the method comprising performing an assay method as described herein on a sample obtained from the athlete to ascertain if the sample comprises a ligand sufficient to activate a steroid hormone receptor and cause a change in a physical property of the reporter construct, wherein a change in a physical property of the reporter construct provides information about the doping status of the athlete.

In a further aspect of the present invention there is provided an article of manufacture for screening a sample for the presence of a ligand, which ligand is capable of eliciting a steroid hormone genomic response, the article of manufacture comprising a test kit as described herein together with instructions for how to detect the presence of a ligand in the sample.

In a further aspect of the present invention there is provided an article of manufacture for screening a biological sample for the presence of a ligand, which ligand is capable of eliciting a steroid hormone genomic response, the article of manufacture comprising a test kit as described herein together with instructions for how to detect the presence of a ligand in the biological sample.

In a further aspect of the present invention there is provided an article of manufacture for screening a food or a nutritional supplement for the presence of a ligand, which ligand is capable of eliciting a steroid hormone genomic response, the article of manufacture comprising a test kit as described herein together with instructions for how to detect the presence of a ligand in the food or nutritional supplement.

In yet a further aspect of the present invention there is provided an article of manufacture for determining the steroid hormone bioactivity of a sample, the article of manufacture comprising a test kit as described herein together with instructions for detecting the steroid hormone bioactivity in a sample, wherein the presence of bioactive ligands in the sample is indicative of steroid hormone bioactivity of the sample.

In yet a further aspect of the present invention there is provided an article of manufacture for determining the steroid hormone bioactivity of a clinical specimen, the article of manufacture comprising a test kit as described herein together with instructions for detecting the steroid hormone bioactivity in a clinical specimen, wherein the presence of bioactive ligands in the clinical specimen is indicative of steroid hormone bioactivity of the clinical specimen.

In yet a further aspect of the present invention there is provided an article of manufacture for determining the steroid hormone bioactivity of an environmental sample, the article of manufacture comprising a test kit as described herein together with instructions for detecting the steroid hormone bioactivity in an environmental sample, wherein the presence of bioactive ligands in the environmental sample is indicative of steroid hormone bioactivity of the environmental sample.

In yet a further aspect of the present invention there is provided an article of manufacture for determining doping in an athlete, the article of manufacture comprising a test kit as described herein together with instructions for detecting the presence of a ligand in a sample derived from the athlete, wherein the presence of the ligand in the sample is indicative of doping in the athlete.

### BRIEF DESCRIPTION OF THE FIGURES

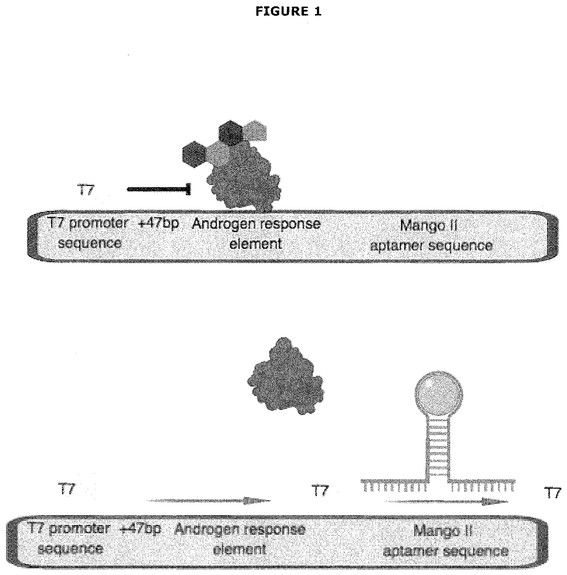

**Figure 1** **shows** a schematic representation of androgen response element (ARE) mediated inhibition of T7 RNA polymerase mediated transcription of a reporter construct comprising Mango II aptamer sequence under control of the T7 promoter.

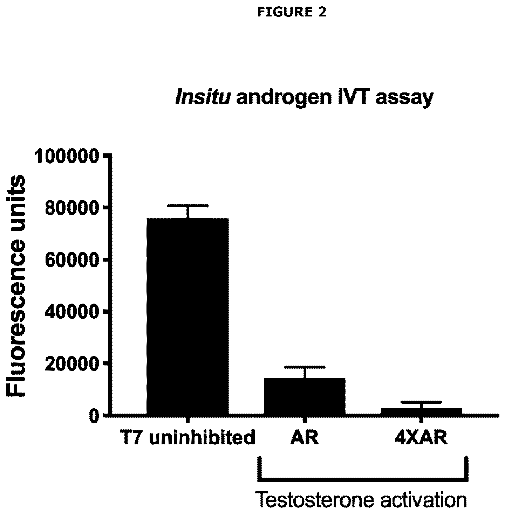

**Figure 2** **shows** AR reduces T7-mediated transcription of RNA Mango II aptamer. *In vitro* transcription reactions were assembled using a T7 promoter-ARE-RNA Mango II aptamer DNA template. Reactions were assembled in 0.5 mL Eppendorf tubes, and initiated by addition of 11.5 ng/mL testosterone. After 2 hours incubation, RNA Mango II aptamer was measured with the addition of TO1-biotin (TO1-B), and increased fluorescence detected using standard fluorimetry. Black column-control reaction with no AR added, Grey columns-reaction with AR added at 100 ng/reaction or 400 ng/reaction.

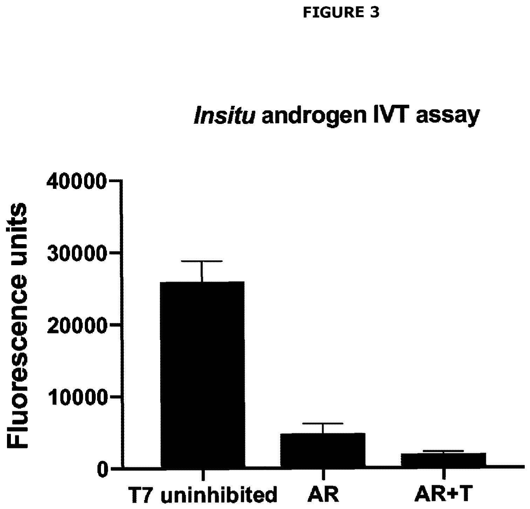

**Figure 3** **shows** AR reduces T7-mediated transcription of RNA Mango II aptamer. *In vitro* transcription reactions were assembled using a T7 promoter-ARE-RNA Mango II aptamer DNA template. Reactions were assembled in 0.5 mL Eppendorf tubes. After a 2 hour incubation, RNA Mango II aptamer was measured with the addition of TO1-B, and increased fluorescence detected using a standard fluorimeter. Black column-control reaction with no AR added, Grey column- reaction with AR added at 100n g/reaction. Dark grey column - reaction with AR added at 100 ng/reaction and activated with 11.5n g/mL testosterone.

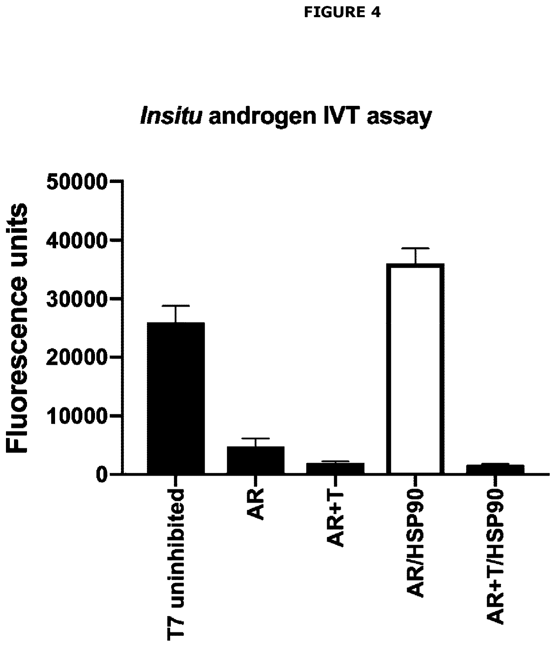

**Figure 4** **shows** HSP90 blocks AR inhibition of T7-mediated RNA Mango II aptamer expression. *In vitro* transcription reactions were assembled using the T7 promoter-ARE-RNA Mango II aptamer DNA template. Reactions were assembled in 0.5 mL Eppendorf tubes. After a two-hour incubation, RNA Mango II aptamer was measured with the addition of TO1-B, and increased fluorescence detected using a standard fluorimeter. Black column-control reaction with no AR added, Grey column- reaction with AR added at 100ng/reaction. Dark grey column - reaction with AR added at 100 ng/reaction and activated with 11.5 ng/mL testosterone. Light grey column- reaction with AR added at 100 ng/reaction and HSP90 at 200 ng/reaction. Darkest grey column- reaction with AR added at 100 ng/reaction and HSP90 at 200 ng/reaction and testosterone added at 11.5 ng/mL.

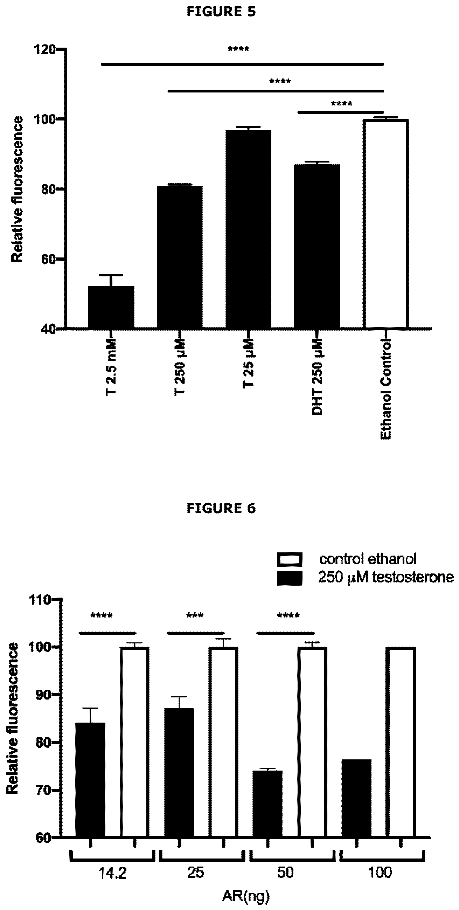

**Figure 5** **shows** testosterone-activated AR dose-dependently reduces T7-mediated expression of RNA aptamer, Mango II. The reactions were assembled as described in the text. For these reactions, T7-generated RNA Mango II was evidenced by the ethanol control. An ethanol control is included as this is the diluent for the steroids, testosterone (T) and dihydrotestosterone (DHT). The fluorescence output generated for the ethanol control was arbitrarily assigned 100%, and used as the reference for the ligand-activated reactions. Testosterone at decreasing concentration (across the range of 2.5mM to 25 µM) was added to activate AR, or alternatively DHT was added at 250 µM. Both T and DHT are endogenous androgens and potent activators of AR. ****p<0.001 (vs ethanol) one-way ANOVA with Dunnett's multiple comparison test.
**Figure 6** **shows** titration of AR directly influences ΔMango II. The reactions were assembled as described in the text (e.g. see Table 7). For these reactions, T7-generated RNA Mango II was evidenced by the ethanol control (grey columns). An ethanol control is included as this is the diluent for testosterone (T). The fluorescence output generated for the ethanol control was arbitrarily assigned 100%, and used as the reference for the 250 µM testosterone-activated reactions (black columns). Decreasing the concentration of AR from 50 ng to 25 ng to 14.2 ng maintained a significant level of AR blockade (n=5), however the effect size of T7 inhibition decreased relative to 50ng. Increasing AR concentration to 100ng (n=2) had no further effect on T7 inhibition. ****p<0.001, ***p=0.002 (vs ethanol) one-way ANOVA with Dunnett's multiple comparison test.

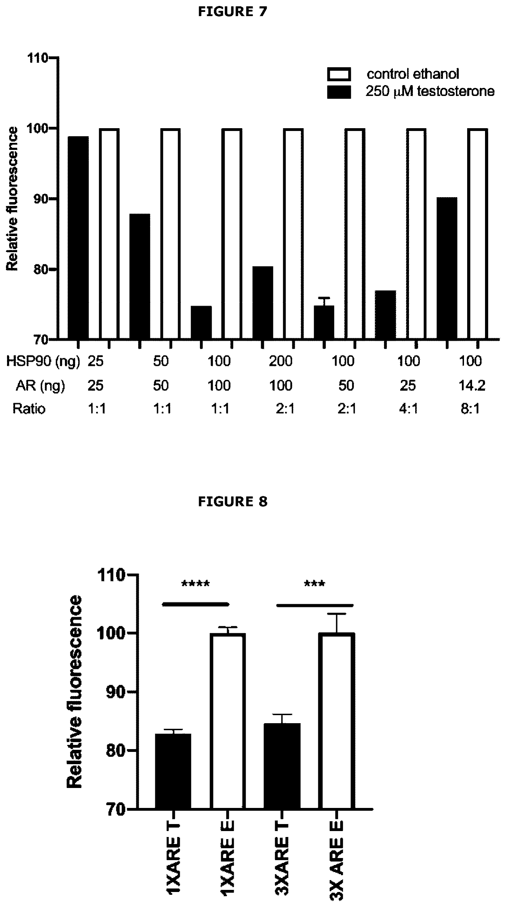

**Figure 7** **shows** the HSP90:AR ratio influences ΔMango II. The reactions were assembled as described in the text (see Table 8). For these reactions, T7-generated RNA Mango II was evidenced by the ethanol control (grey columns). The fluorescence output generated for the ethanol control was arbitrarily assigned 100%, and used as the reference for the 250 µM testosterone-activated reactions (black columns). The results show that a 1:1 ratio is effective only as AR concentrations increase, with greatest ΔMango II recorded when AR concentration is 100ng. A 2:1 ratio was effective if AR concentration was 50 ng or 100 ng, while a 4:1 ratio allowed AR to operate even with lower AR concentrations while an 8: 1 ratio showed decreased effect size on ΔMango II.
**Figure 8** **shows** a single ARE site is strong enough to reduce T7 transcription. The reactions were assembled as described in the text, except for the DNA templates differed. For one set of reaction, a DNA template was used that encoded just a single ARE, while for the second set of reactions, the original 3XARE DNA template was used (Sequences shown in Table 2). For these reactions, T7-generated RNA Mango II was evidenced by the ethanol control (grey columns). The fluorescence output generated for the ethanol control was arbitrarily assigned 100% and used as the reference for the 250 µM testosterone-activated reactions (black columns). The results show that a single ARE is as effective as the 3XARE (****p<0.0001, ***p=0.002).

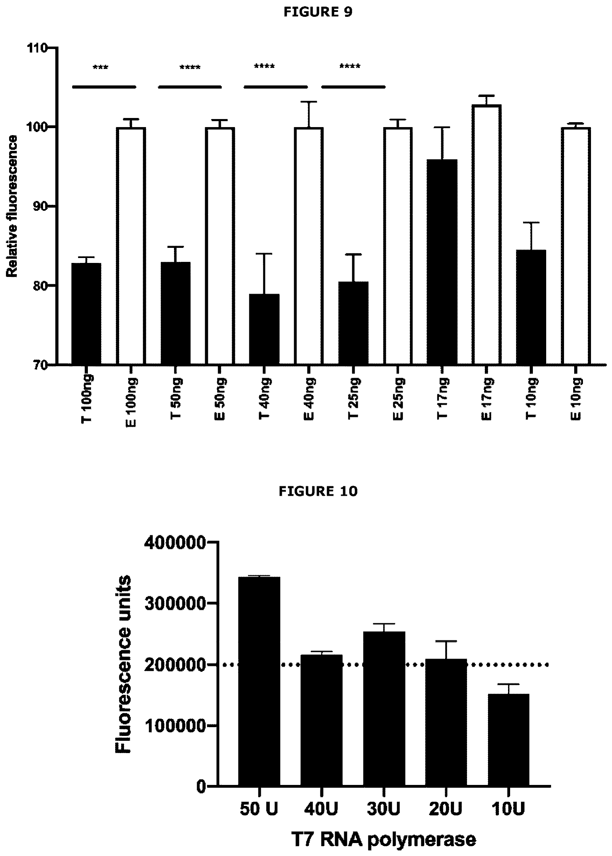

**Figure 9** **shows** decreasing DNA template concentration reduces ΔMango II. The reactions were assembled as described in the text. For these reactions, T7-generated RNA Mango II was evidenced by the ethanol control (grey columns). The fluorescence output generated for the ethanol control was arbitrarily assigned 100% and used as the reference for the 250 µM testosterone-activated reactions (black columns). The results show that as DNA template concentration dropped below 25ng per reaction there was a loss in the ability to detect ΔMango II (****p<0.0001, ***p=0.003).
**Figure 10** **shows** titrating T7 units from 50U to 10U decreases fluorescence below a detection of change threshold. T7 RNA polymerase was titrated such that 50U to 10U of enzyme was added per reaction. The reaction only consisted of the T7 RNA polymerase, the DNA template and the reaction buffer. Notably, at 50U the Mango II generated from the reaction resulted in a fluorescence readout of ~340000. This reduced nicely to ~200000 when 40U of enzyme was added. Any further dilution did not generate a measurable change in fluorescence indicating that ~ 200000 fluorescence units is the threshold for this reaction.

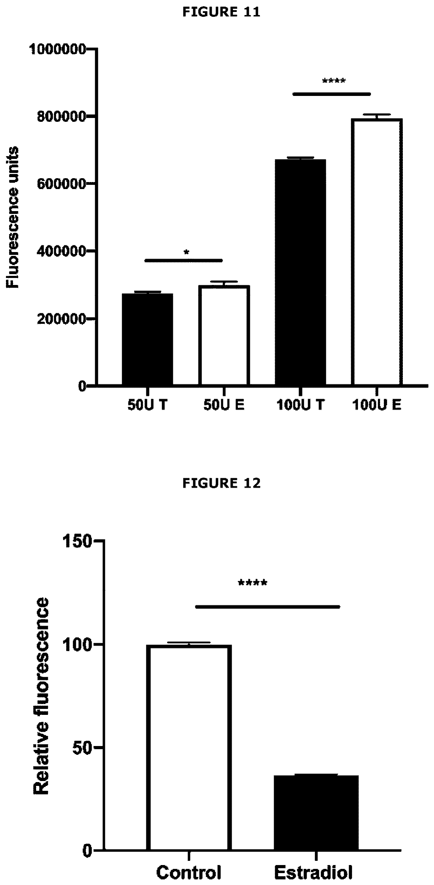

**Figure 11** **shows** the threshold for ΔMango II detection. Reactions were established with 50U or 100U of T7 RNA polymerase, DNA template, reaction buffer, 50 ng AR, 100 ng HSP90, and 250 µM testosterone (or ethanol as control). Data shows that when 50U T7 enzyme was added, there was ability to detect ΔMango II however the effect size is relatively small, albeit significant (n=5, *p=0.0155 one way ANOVA with Sidaks post-hoc test). When 100U of T7 enzyme was added, ΔMango II was easily detected with a greater effect size (n=5, ****p<0.0001). The threshold for ΔMango II detection is ~200000. If output falls below this, interpretation of test results will be difficult.
**Figure 12** **shows** A single ERE site is strong enough for estradiol-activated ERα to reduce T7 transcription. The reactions were assembled as described in the text. For these reactions, T7-generated RNA Mango II is evidenced by the ethanol control. An ethanol control is included as this is the diluent for the steroid hormone, estradiol (E2). The fluorescence output generated for the ethanol control was arbitrarily assigned 100%, and used as the reference for the estradiol-activated reactions. Estradiol at 5uM was added to activate ERα. n=3, ****p<0.001 (vs ethanol) One-way ANOVA with Dunnett's multiple comparison test.

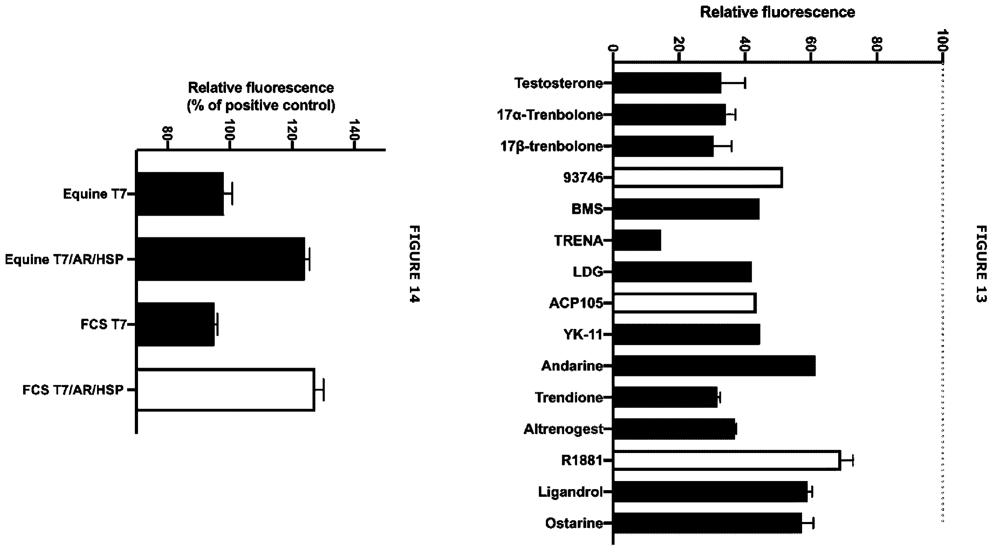

**Figure 13** **shows** the AR/HSP90-ARE assay is able to detect a range of AAS and SARMs. Testosterone (250 µM) was used as the positive reference while ethanol was used as the negative reference and activity set at 100% (dotted green line). All data is normalized to the ethanol control. The class of androgenic molecules is described in the text (Table 10).
**Figure 14** **shows** RNA polymerase activity is not affected by serum. The reactions were assembled with DNA template (100 ng), buffer, nuclease-free water and for AR reactions, AR (50 ng), HSP90 (100 ng). Equine serum or FCS (10 µl) were added before the reaction was initiated with the addition 100U (or equivalent) T7 RNA polymerase. The reactions were held at 37°C for 150 mins before Mango II RNA aptamer was detected with TO1-biotin (100 nM). The data shows that the presence of equine serum or FCS had no effect on T7-generation of Mango II.

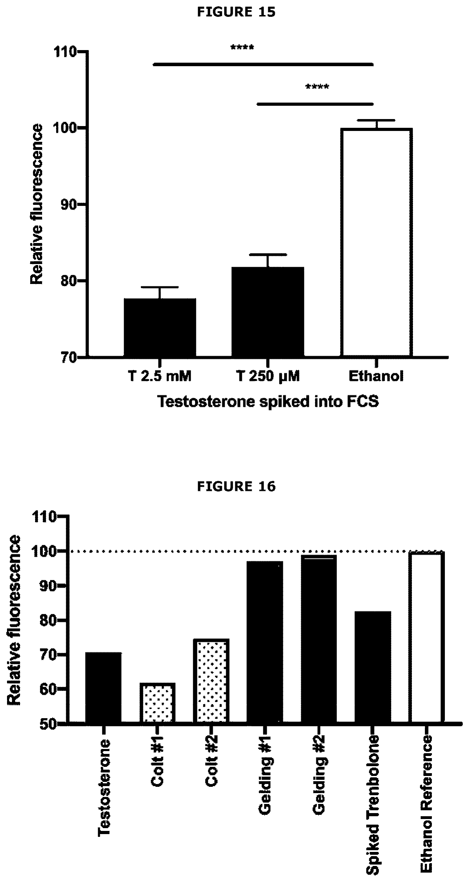

**Figure 15** **shows** detection of testosterone in serum samples. Reactions were assembled as described in text with the exception that 13 µl FCS was added that had been spiked with testosterone. Ethanol-spiked FCS was used as the non-activating AR control and the T7 activity was arbitrarily set at 100%. Testosterone showed a dose-dependent suppression of T7-generated Mango II, with a ΔMango II greater for the higher testosterone concentration. p<0.0001 versus ethanol, one-way ANOVA with Sidak's post-hoc comparison.
**Figure 16** **shows** detection of androgenic activity in urine samples. Reactions were assembled as described in text. Ethanol was used as the negative (vehicle) control for AR activation, while testosterone (250 µM) was used as the positive control. Colt #1 and Colt #2 represent urine samples obtained from two different young male racehorses. Gelding #1 and gelding #2 represent urine samples obtained from two different male castrated horses. Spiked trenbolone represents a gelding urine sample that had been spiked with trenbolone before the deconjugation and extraction steps. T7 activity measured for the ethanol control was arbitrarily set at 100%. Testosterone showed suppression of T7-generated Mango II that was also seen with the colt urine samples and the spiked trenbolone urine sample, however very little suppression of T7 activity was measured for the gelding samples. Geldings have had their testes removed and as these organs are the major source of testosterone production in males, endogenous androgen levels would be expected to be low.

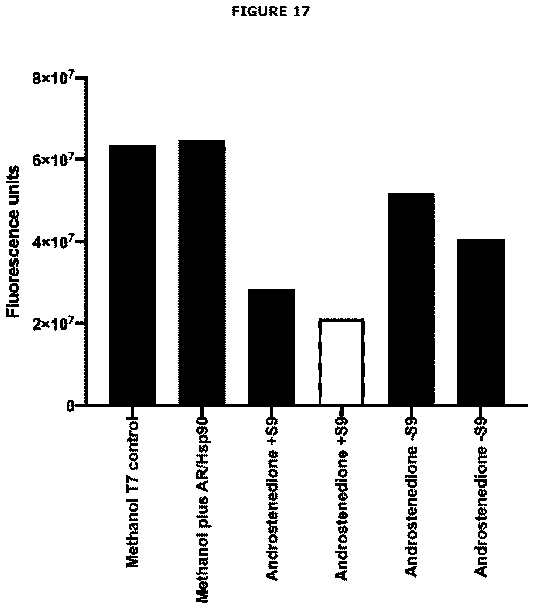

**Figure 17** **shows** pro-hormone detection using the SHR/SRE assay. Androstenedione was preincubated with S9 liver fraction before the liver S9 fraction reaction was extracted for steroids. The extracted samples were then tested for androgenic activity. Data shows that methanol control represents full T7 activity, that is not affected when AR/HSP90 is added in the absence of an androgen. When the Androstenedione/S9 extracted sample was tested there was strong reduction in fluorescence readout (n=2 independent steroid metabolism/extraction reactions) compared to Androstenedione (n=2 independent no NAD S9 fraction/extraction reactions).

### GENERAL DEFINITIONS

Unless specifically defined otherwise, all technical and scientific terms used herein shall be taken to have the same meaning as commonly understood by one of ordinary skill in the art (for example, in immunology, immunohistochemistry, protein chemistry, molecular genetics, synthetic biology and biochemistry).

It is intended that reference to a range of numbers disclosed herein (e.g. 1 to 10) also incorporates reference to all related numbers within that range (e.g. 1, 1.1, 2, 3, 3.9, 4, 5, 6, 6.5, 7, 8, 9 and 10) and also any range of rational numbers within that range (for example 2 to 8, 1.5 to 5.5 and 3.1 to 4.7) and, therefore, all sub-ranges of all ranges expressly disclosed herein are expressly disclosed. These are only examples of what is specifically intended and all possible combinations of numerical values between the lowest value and the highest value enumerated are to be considered to be expressly stated in this application in a similar manner.

The term "and/or", e.g., "X and/or Y" shall be understood to mean either "X and Y" or "X or Y" and shall be taken to provide explicit support for both meanings or for either meaning.

The term "a" or "an" refers to one or more than one of the entity specified; for example, "a receptor" or "a nucleic acid molecule" may refer to one or more receptor or nucleic acid molecule, or at least one receptor or nucleic acid molecule. As such, the terms "a" or "an", "one or more" and "at least one" can be used interchangeably herein.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

### SELECTED DEFINITIONS

For the purposes of the present invention, the following terms shall have the following meanings.

The term "test kit" as used herein refers to an article of manufacture comprising various components to perform the assays and methods according to the inventions descibed herein.

The term "steroid hormone receptor" or "SHR" as used herein refers to a protein or polypeptide, including recombinant polypeptides that selectively binds to a ligand, which ligand is capable of activating the steroid hormone receptor, and includes, without limitation, an androgen receptor, an estrogen receptor, a progesterone receptor, a mineralocorticoid receptor and a glucocorticoid receptor. Typically, a steroid hormone receptor comprises a ligand binding domain, an activation domain and a deoxyribonucleic acid binding domain. According to this definition, "steroid hormone receptor" may optionally include other cofactors, including (e.g.) heat shock proteins and the like, which help to hold the steroid hormone receptor in a folded and hormone responsive state for activation by a ligand.

The term "steroid hormone receptor cofactor" as used herein refers to one or more cofactors that hold the steroid hormone receptor in an inactive state, until displaced by a ligand, at which point the steroid hormone receptor becomes activated. Examples of steroid hormone receptor cofactors according to the present invention include, without limitation, heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.

The term "ligand" refers generally to any molecule that binds to a receptor, and includes without limitation, a steroid, a polypeptide, a protein, a vitamin, a carbohydrate, a glycoprotein, a therapeutic agent, a drug, a glycosaminoglycan, or any combination thereof. As used herein, "ligand" includes, without limitation, steroid hormones, such as sex hormones including but not limited to estrogens, progestagens, androgens etc, as well as natural and synthetic derivatives and analogs and metabolites thereof, designer steroid hormones, anabolic androgenic steroids, and selective androgen-, progestagen- and estrogen receptor modulators, those that are currently known and those anticipated to be developed or naturally found in biological samples.

The term "receptor-ligand complex" and "activated steroid hormone receptor" as used interchangeably herein to refer to a ligand bound steroid hormone receptor, where the steroid hormone receptor undergoes a structural transformation upon binding the ligand and is then said to be in an activated form. A receptor-ligand complex as described herein includes, without limitation, a dimer of a ligand bound hormone receptor (i.e. (HR-L)₂.

The term "genomic response" as used herein refers to the ability of an activated steroid hormone receptor (or receptor-ligand complex) to selectively bind to its corresponding nucleic acid response element and activate or repress transcription of downstream genes. In the cellular environment, the ligand-bound receptor binds to the nucleic acid response element and switches genes on or off in response to the external stimuli (i.e. presence of a ligand). The test kits, assays and methods according to the present invention have been developed to identify ligands which, in a cellular environment, would elicit a steroid hormone genomic response by providing reporter based frameworks which mimic aspects of cell based systems.

The term "steroid metabolism machinery" as used herein refers to any enzyme, and includes combinations of enzyme, sufficient to convert a ligand from a physiologically inactive form to a physiologically active form or from a physiologically active form to a *more* physiologically active form, or from a physiologically active form to a less physiologically active form, or from a physiologically active form to a physiologically inactive form.

The term "detection means" as used herein refers to any apparatus, equipment or configuration adapted to detect the binding interaction between an activated steroid hormone receptor and nucleic acid response element. Examples of detection means include, but are not limited to, optical methods, spectroscopy, visible spectroscopy, Raman spectroscopy, UV spectroscopy, surface plasmon resonance, electrochemical methods, impedance, resistance, capacitance, mechanical sensing by changes in mass, changes in mechanical resonance, electrophoresis, gel electrophoresis, gel retardation, imaging, fluorescence and fluorescence resonance energy transfer, polymerase chain reaction etc.

The term "nucleic acid sequence" as used herein refers to a deoxyribonucleic acid (DNA) sequence, a ribonucleic acid sequence (RNA), messenger ribonucleic acid (mRNA) and complementary DNA (cDNA), and is comprised of a continuous sequence of two or more nucleotides, also referred to as a polynucleotide or oligonucleotide. The nucleic acid sequence may be single-stranded or double-stranded.

The term "reporter construct" as used herein refers to a nucleic acid sequence encoding a reporter molecule that encodes an RNA whose expression may be assayed; such RNA includes, but are not limited to, fluorophore binding aptamers, or synthetic RNA or mRNA, Additionally, reporter genes may encompass any gene of interest whose expression product may be detected by RNA analysis.

The term "promoter" as used herein is a nucleic acid sequence located proximal to the start of transcription at the 5' end of an operably linked transcribed sequence. The promoter may contain one or more regulatory elements which interact in modulating transcription of an operably linked gene. Examples of promoters according to the present invention include, but are not limited to, T7, T3 and SP6 bacteriophage promoters or initiation sequences. The terms "promoter", "promoter sequence", "initiator sequence" or "initiation sequence" may be used interchangeably throughout this specification to mean the same thing.

The term "operably linked" as used herein describes two macromolecular elements arranged such that modulating the activity of the first element induces an effect on the second element. In this manner, modulation of the activity of a promoter element may be used to alter and/or regulate the expression of an operably-linked reporter construct. For example, the transcription of a reporter construct that is operably-linked to a promoter element is induced by factors that "activate" the promoter's activity; transcription of a reporter construct that is operably-linked to a promoter element is inhibited by factors that "block" the promoter's activity. Thus, a promoter region is operably-linked to the reporter construct if transcription of such a reporter construct is influenced by the activity of the promoter.

The term "expression" as used herein refers to the process by which the information encoded within a gene is expressed. If the gene encodes a protein, expression involves both transcription of the DNA into mRNA, the processing of the mRNA (if necessary) into a mature mRNA product, and translation of the mature mRNA into protein. A nucleic acid molecule, such as a deoxyribonucleic acid (DNA) or gene is said to be "capable of expressing" a polypeptide (or protein) if the molecule contains the coding sequences for the polypeptide and the expression control sequences which, in the appropriate host environment, provide the ability to transcribe, process and translate the genetic information contained in the DNA into a protein product, and if such expression control sequences are operably-linked to the nucleotide sequence that encodes the polypeptide.

The term "sample" as used herein refers to any sample for which it is desired to test for the presence of a ligand. The terms "sample" and "test sample" are used interchangeably in this specification.

The term "relative potency" or "RP" as used herein refers to the multiplier of biological activity exhibited by a test compound relative to a reference compound, where the biological activity is defined by the ability of the compound to bind to and activate a steroid hormone receptor (e.g.) as measured using the assays and test kits described herein. Where Relative Potency is > 1, the test compound is more potent in terms of its biological activity as compared to the reference compound; where Relative Potency is < 1, the test compound is less potent in terms of its biological activity as compared to the reference compound; and where Relative Potency =1, the test and reference compounds are equally potent in terms of their biological activities.

The term "activation factor" or "AF" as used herein relates to the measure of metabolic conversion of a test compound (e.g.) from a physiologically inactive state to a physiologically active state or from a less physiologically active state to a more physiologically active state. An activation factor >1 means that the test compound has undergone metabolic conversion to a more physiologically active state in the presence of metabolic machinery in the assay.

The term "reference threshold" or "reference standard" may be used interchangeably to means the level of assay activity measured (e.g.) in the absence of a test sample, or in the absence of test sample and steroid hormone receptor. In certain examples according to the inventions described herein, the reference threshold or reference standard is determined using ethanol in place of test sample. The reference threshold is intended to establish any baseline activity or signal of the assay in the absence of target ligand.

### DETAILED DESCRIPTION

The present invention provides test kits, assays and methods useful for screening a sample for the presence of a ligand capable of activating a steroid hormone receptor and eliciting a steroid hormone genomic response.

In certain examples according to the present invention, the test kits, assays and methods described herein are useful for determining the hormone status of a subject, for example, by measuring the androgenic and/or estrogenic activity of a sample obtained from the subject. This information may then be used to determine, for example, whether the subject has, or is at risk for developing, cancer, or for investigating endocrine issues associated with ageing such as, for example, menopause, or for evaluating the efficacy of hormone replacement therapy or hormone inhibitory therapy.

In other examples according to the present invention, the test kits, assays and methods described herein are useful for screening foods and health food supplements for banned additives or for natural activators that could be harmful to health including, but not limited to, phytoestrogens that could promote hormone sensitive cancers.

### Enzyme Mediated Activity Assays & Test Kits

The assays according to the present invention, on which the test kits and methods described herein are based, are fundamentally activity based assays which work on the principle of steroid hormone receptor activation through binding of a ligand derived from a sample to be tested. Activation of a steroid hormone receptor occurs when a ligand binds to the receptor and induces a conformational change in the tertiary structure of the protein, meaning that the receptor-ligand complex (also referred to herein as an 'activated steroid hormone receptor') is then able to bind to a nucleic acid response element and influence RNA polymerases and elicit a so-called 'genomic response'. In other words the ability to up- or down-regulate expression of genes from the genome of the cell which may then lead to a physiological effect. It is this binding interaction between the activated steroid hormone receptor and nucleic acid response element that is measured by the test kits, assays and methods described herein, as a proxy to detect the presence of a ligand having *steroid-, or steroid-like* activity in a sample under investigation.

Importantly, this means the test kits and assays according to the present invention have the ability to detect steriod hormone bio/activity elicited by ligands of unknown structure such as (e.g.) 'designer drugs'. Historically, this has not been possible since conventional laboratory testing equipment, such as gas/liquid chromatography and mass spectrometry, requires prior knowledge of the structure of the molecule being investigated.

Previous approaches to detection of steroid hormone bio/activity has involved yeast and mammalian cell reporter assays (e.g. refer to Cooper et al. (2013) Sensors 13:2148-2163). Such assays have well-recognised limitations such as the need for (e.g.) specialised equipment and expertise to maintain living cell cultures. The development of cell-free assays modeled on molecular frameworks that largely mimic cell-based systems has generated *in vitro* assays with enhanced funtionality and improved assay sensitivity compared to its cell-based reporter counterpart(s). However, a limitation of the cell-free assays is the requirement to use multi-subunit holoenzyme polymerases, such as RNA Polymerase II. The recombinant production of RNA polymerase II is extremely difficult to achieve with variable reproducibility, and as a consequence, the holoenzyme is typically made available by using nuclear extracts where all the components exist and come together at the promoter sequence. However, preparing nuclear extracts from eukaryotic cells is expensive to manufacture because of the need for costly cell growth media and the technical expertise required to enrich nuclear materials.

To address this limitation, Applicants have now developed cell-free assays which are based on *in vitro* transcription reactions involving more readily available single polypeptide polymerases.

In developing these next generation assays, Applicants identified that single polypeptide DNA-dependent RNA polymerases derived from (e.g.) bacteriophage infected prokaryote cells were able to express reporter construct read-outs. Refer to Examples 1-2, read in conjunction with Figures 1-16.

Importantly, the use of recombinant, commercially available bacteriophage polymerases replaces the need for expensive-to-manufacture nuclear extracts.

Accordingly, in an aspect of the present invention there is provided a test kit for screening a sample for the presence of a ligand capable of eliciting a steroid hormone genomic response, the test kit comprising:
(i) a steroid hormone receptor that is capable of forming a ligand-receptor complex with a ligand from the sample; and
(ii) a nucleic acid molecule comprising:
   (a) a polymerase promoter sequence;
   (b) a response element that is capable of being bound by the ligand receptor complex; and
   (c) a reporter construct
   where the response element (b) is located between the promoter sequence (a) and the reporter construct (c), and (a), (b) and (c) are operably linked; and
(iii) a single polypeptide polymerase
wherein, the presence of a ligand in the sample is detected by measuring a reduction or inhibition in transcription of the reporter construct caused by binding of the ligand-receptor complex to the response element when the sample is combined with the test kit.

In an example of the present invention, the test kit further comprises nucleotide triphosphates (NTPs).

In yet another aspect of the present invention there is provided an assay method for detecting a ligand in a sample which ligand is capable of eliciting a steroid hormone genomic response, the assay method comprising the steps of:
(i) contacting a sample with:
   (a) a steroid hormone receptor that forms a ligand-receptor complex with a ligand from the sample; and
   (b) a nucleic acid molecule comprising:
      (1) a polymerase promoter sequence;
      (2) a response element that is bound by the ligand-receptor complex; and
      (3) a reporter construct
      where the response element (b) is located between the promoter sequence (a) and the reporter construct (c), and (a), (b) and (c) are operably linked;
   (c) a single polypeptide polymerase; and
   (d) nucleoside triphosphates; and
(ii) measuring a reduction or inhibition in transcription of the reporter construct caused by binding of the ligand-receptor complex to the response element,
wherein, a measured reduction or inhibition in transcription of the reporter construct reflects detection of a ligand in the sample.

An important advantage conferred by the test kits and assay methods described herein is a significant reduction in the molecular complexity otherwise present in the cellular environment of yeast and mammalian cell reporter assays, or molecular complexity created through use of nuclear extracts for cell-free assays such as those described in WO2018/088852 which require RNA Polymerase II for performance. Reduced molecular complexity significantly enhances assay specificity by limiting non-specific activation of the hormone response element by steroid hormone receptor(s) in the absence of target ligand. It is understood that hormone response elements (e.g. androgen response element) are not highly selective for their complimentary receptor (e.g. androgen receptor), and indeed may be activated by other steroid hormone receptors that may be present within the cell or nuclear extract. For example, in the case of androgen response element, other non-androgen receptors in the Group II hormone receptor class such as progesterone receptor A, progesterone receptor B, glucocorticoid receptor and/or mineralocorticoid receptor could potentially activate the ARE. This in turn creates reduced assay specificity.

Indeed, the absence of molecular complexity created by a cellular environment or nuclear extract means that the test kits and assay methods described herein are highly selective for detection of their target ligands. Further, the ability to easily switch out (e.g.) a steroid hormone receptor and/or hormone response element creates versatility in the test kits and assay methods described herein for detection of other target ligands of interest.

Another important advantage conferred by the test kits and assay methods described herein is the unique ability to stoichiometrically define a biological reaction. For example, by precisely controlling the molecular relationship(s) between both essential and non-essential assay components, assay sensitivity may be significantly enhanced for the detection of a target ligand. In other words, the test kits and assay methods described herein may be configured to measure an *optimum* number of binding interactions between activated ligand-hormone receptor complexes and hormone response elements present within reporter constructs. In contrast, it is difficult, if not impossible, to replicate the same degree of control for cell-based reporter assays which have been configured to detect the presence of a ligand, since the total copy number of the gene or nucleic acid sequence encoding (e.g.) recombinant receptor and/or nucleic acid response element cloned into the cell cannot be predicted or controlled with any accuracy. It is also difficult to control for cell-free reporter assays based on nuclear extracts where it is difficult to determine exact amount of RNA polymerase II subunits present, cofactors, and other non-essential proteins.

These and other considerations are documented by the Examples which follow. By way of illustration, refer to Example 2 when read in conjunction with Figures 6-9. Specifically, in a prototype assay involving detection of androgenic ligands (e.g. Testosterone) ('Androgen Assay Prototype 2') Applicants demonstrate that activated androgen receptor (AR) is most effective at binding to its complimentary hormone response element (ARE; located within the nucleic acid sequence as defined herein) and blocking T7 mediated expression of a reporter construct when (i) the AR:nucleic acid ratio is ≥ 7.2, and (ii) the concentration of nucleic acid in the reaction mix is ≥ 0.789 nM.

Accordingly, in a further example according to the test kits, assays and methods described herein, the ratio of steroid hormone receptor to nucleic acid is between about 7:1 and about 10:1, and includes without limitation, 7.0:1, 7.1:1, 7.2:1, 7.3:1, 7.4:1, 7.5:1, 7.6:1, 7.7:1, 7.8:1, 7.9:1, 8.0:1, 8.1:1, 8.2:1, 8.3:1, 8.4:1, 8.5:1, 8.6:1, 8.7:1, 8.8:1, 8.9:1, 9.0:1, 9.1:1, 9.2:1, 9.3:1, 9.4:1, 9.5:1, 9.6:1, 9.7:1, 9.8:1, 9.9:1 and 10.0:1.

The skilled person would further appreciate that the molecular stoichiometry between the steriod hormone receptor and nucleic acid comprising the hormone receptor response element may vary depending on the component parts of the test kit/assay, as well as the nature of the sample to be tested. For example, the test kit/assay may by configured to detect ligands which bind to different steroid hormone receptors including, but not limited to, androgen receptor, estrogen receptor alpha, estrogen receptor beta, progesterone receptor A, progesterone receptor B, mineralocorticoid receptor and glucocorticoid receptor, where the ratio between the steroid hormone receptor and its complimentary nucleic acid/response element may be (e.g.) between about 1:1 and about 20:1.

Accordingly, in yet another example according to the test kits and assay methods described herein:
(i) the test kit comprises an androgen receptor, and the ratio of androgen receptor to nucleic acid comprising an androgen response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(ii) the test kit comprises an estrogen receptor, and the ratio of estrogen receptor to nucleic acid comprising an estrogen response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(iii) the test kit comprises a progesterone receptor, and the ratio of progesterone receptor to nucleic acid comprising a progesterone response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(iv) the test kit comprises a mineralocorticoid receptor, and the ratio of mineralocorticoid receptor to nucleic acid comprising a mineralocorticoid response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1; and
(v) the test kit comprises a glucocorticoid receptor, and the ratio of glucocorticoid receptor to nucleic acid comprising a glucocorticoid response element is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1.

Notwithstanding the above considerations, care must be taken not to saturate the assay with too much receptor, since this in itself may create thermodynamic or kinetic barriers that prevent optimal binding of a ligand-receptor complex to its complimentary response element. According to the disclosure provided herein, the skilled person could undertake routine experimentation to titrate an optimized conentration/range of steroid hormone receptor for performance of the test kits and assay methods described herein (e.g. refer to Example 2.3 which follows).

As previously mentioned, Applicants have further determined that the steroid hormone receptor retains *some* capacity to bind to and activate its corresponding nucleic acid response element in the absence of a ligand specific for its steroid hormone receptor. This phenomena is sometimes referred to as auto-activation of the nucleic acid response element. While, by virtue of the reduced molecular complexity, the level of auto-activation is significantly diminished in the test kits and assays described herein, it may be desirable to determine a reference threshold (i.e. baseline signal as a result of auto-activation of the response element) in the absence of ligand to assist with a determination of absolute assay signal/readout in the presence of a sample containing a target ligand.

Accordingly, in another example according to the test kits and assay methods described herein, the reduction or inhibition in transcription of the reporter construct is measured relative to a reference threshold.

In yet another example according to the test kits and assay methods described herein, reduction or inhibition in transcription of the reporter construct is measured relative to a reference threshold as determined by measuring transcription of the reporter construct in the absence of a test sample.

In yet a further example according to the test kits and assay methods described herein, reduction or inhibition in transcription of the reporter construct is measured relative to a reference threshold as determined by measuring transcription of the reporter construct in the absence of a test sample and in the absence of receptor.

In a parallel approach to enhance assay specificity and performance, the test kit and assay methods described herein may be modified to include at least one steroid hormone receptor cofactor. The primary purpose of the cofactor is to hold the steroid hormone receptor in an inactive conformation, thereby preventing it from binding to and activating the hormone response element in the absence of a target ligand.

When the test kit is contacted (or combined) with a test sample, the presence of a ligand causes displacement of the cofactor and the ligand-bound receptor is then free to form a complex with a second ligand-bound receptor and the hormone response element.

Accordingly, in another example according to the test kits and assay methods described herein, the test kit or assay method further comprises at least one steroid hormone receptor cofactor.

In a related example, the steroid hormone receptor cofactor includes, without limitation, a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.

In a further related example according to the test kits and assay methods described herein, the test kits or assay methods further comprise heat shock protein 90.

It will, however, be appreciated by a person skilled in the art that the presence of steroid hormone receptor cofactor is not an essential feature of the test kits, assays and methods described herein. This is because an assay result may still be achieved in the absence of cofactor. For example, the data presented in Example 1/Figure 4 illustrates there was *still* a measurable difference in signal between the ligand and non-ligand assay result (i.e. AR/T *versus* AR) in the absence of heat shock protein 90.

Indeed, there are further approaches in which to minimise the amount of signal generated by auto-activation of the hormone response element by non-liganded receptor, for example, by modifying the temperature at which an assay method is performed.

Applicants have observed a differential in the binding affinty/kinetics between ligand bound and non-ligand bound steroid hormone receptor for its nucleic acid response element. Accordingly, the test kits, assays and methods described herein may be performed at a temperature, or in a temperature range, that preferentially measures activation of a hormone response element by ligand-bound receptor over non-ligand bound receptor, thereby minimising any background signal generated by non-ligand bound receptor.

Accordingly, in yet another example according to this aspect of the present invention, performance of the test kit or assay method is carried out in a temperature range from about 25 °C to about 42 °C, and preferably from about 35 °C to about 37 °C.

The term "a temperature range from about 25 °C to about 42 °C" is intended to include any temperature from 25 °C to 42 °C and without limitation includes 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C and 42 °C. The skilled person would recognise that temeratures in the decimal point range may also be used. To further illustrate this point, "in a temperature range from about 35 °C to about 37 °C" includes, without limitation, 35.0 °C, 35.1 °C, 35.2 °C, 35.3 °C, 35.4 °C, 35.5 °C, 35.6 °C, 35.7 °C, 35.8 °C, 35.9 °C, 36.0 °C, 36.1 °C, 36.2 °C, 36.3 °C, 36.4 °C, 36.5 °C, 36.6 °C, 36.7 °C, 36.8 °C, 36.9 °C and 37.0 °C.

The Applicants further discovered that the stoichiometric relationship between the cofactor and steroid hormone receptor may be manipulated to further enhance assay sensitivity. For example, according to the Androgen Assay Prototype 2 described in Example 2, it was determined by the Applicants that AR is most effective at being activated by an AR-specific ligand and binding to ARE when the HSP90:AR ratio is between 1.22 and 4.88.

Accordingly, in a further example according to the test kits and assay methods described herein, the ratio of HSP90 to steroid hormone receptor is defined as between about 1:1 to about 5:1. This includes, without limitation, a ratio of HSP90 to steroid hormone receptor that is defined as 1.0:1, 1.1:1, 1.2:1, 1.3:1, 1.4:1, 1.5;1, 1.6:1, 1.7:1, 1.8:1, 1.9:1, 2.0:1, 2.1:1, 2.2:1, 2.3:1, 2.4:1, 2.5;1, 2.6:1, 2.7:1, 2.8:1, 2.9:1, 3.0:1, 3.1:1, 3.2:1, 3.3:1, 3.4:1, 3.5;1, 3.6:1, 3.7:1, 3.8:1, 3.9:1, 4.0:1, 4.1:1, 4.2:1, 4.3:1, 4.4:1, 4.5;1, 4.6:1, 4.7:1, 4.8:1, 4.9:1 or 5.0:1.

The skilled person would, however, appreciate that the molecular stoichiometry between the steriod hormone receptor cofactor and steroid hormone receptor may vary depending on the composition of the test kit/assay. For example, the test kit/assay may be configured to detect ligands which bind to an estrogen receptor, including estrogen receptor alpha or estrogen receptor beta, and the ratio between the estrogen receptor cofactor and estrogen receptor may be (e.g.) between about 1:1 and about 20:1.

Accordingly, in yet another example according to the test kits and assay methods described herein:
(i) the test kit comprises an androgen receptor, and the ratio of androgen receptor cofactor to androgen receptor is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(ii) the test kit comprises an estrogen receptor, and the ratio of estrogen receptor cofactor to estrogen receptor is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(iii) the test kit comprises a progesterone receptor, and the ratio of progsterone receptor cofactor to progesterone receptor is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1;
(iv) the test kit comprises a mineralocorticoid receptor, and the ratio of mineralocorticoid receptor cofactor to mineralocorticoid receptor is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1; and
(v) the test kit comprises a glucocorticoid receptor, and the ratio of glucocorticoid receptor cofactor to glucocorticoid receptor is between about 1:1 and about 20:1, including without limitation 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1 or 20:1.

The information in Table 9 of Example 4 summarises key considerations related to the molecular stoichiometry argument when considering the composition of the test kits and assay methods described herein, which is supported by the data accompanying Examples 1-3, in particular.

An assay reaction mix according to the present invention will typically contain between 10 uL and 50 uL total volume. According to the information summarised in Table 9, this means:
(i) the concentration of steroid hormone receptor should be held within a range defined by about 10 nM to about 60 nM which includes, but is not limited to, 10, 11, 12, 13, 14, 15, 16, 17, 18, 19, 20, 21, 22, 23, 24, 25, 26, 27, 28, 29, 30, 31, 32, 33, 34, 35, 36, 37 38, 39, 40, 41, 42, 43, 44, 45, 46, 47, 48, 49, 50, 51, 52, 53, 54, 55, 56, 57, 58, 59 or 60 nM of steroid hormone receptor;
(ii) the concentration of nucleic acid molecule comprising a hormone response element should be held in a range defined by about 0.70 nM to about 3.5 nM which includes, but is not limited to, 0.70, 0.80, 0.90, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2 or 3.3 nM of nucleic acid molecule; and
(iii) where present, the concentration of heat shock protein 90 should be held in a range defined by about 40 nM to about 60 nM which includes, but is not limited to, 40, 41, 42, 43, 44, 45, 46, 47, 48, 49, 50, 51, 52, 53, 54, 55, 56, 57, 58, 59 or 60 nM of heat shock protein 90.

A further consideration is the concentration/amount of enzyme, where optimal assay results were achieved when ~50-100 enzyme units (U) were employed in the reaction mixes assuming an absolute baseline threshold activity of about 200,000 U. Refer to Figs 10 and 11.

In yet another example according to the test kits and assay methods described herein, the reduction or inhibition in transcription is a reduction or inhibition in transcription mediated by a single polypeptide polymerase selected from a bacteriophage RNA polymerase, a virus RNA polymerase, a bacterial RNA polymerase, and a eukaryotic virus RNA polymerase.

In a related example, the polymerase is a bacteriophage RNA polymerase. In a further related example, the promoter sequence is a bacteriophage RNA polymerase initiation sequence.

In yet another example according to the test kits and assay methods described herein, the polymerase is a bacteriophage polymerase selected from the group consisting of DNA-dependent RNA polymerases including T7 RNA polymerase, T3 RNA polymerase and SP6 RNA polymerase.

In another example according to the test kits and assay methods described herein, the bacteriophage polymerase is T7 RNA polymerase.

In another example according to the test kits and assay methods described herein, the promoter sequence is a T7 RNA polymerase initiation sequence.

In a related example, the T7 RNA polymerase initiation sequence comprises a sequence defined by 5'-TAATACGACTCACTATAG-3' (SEQ ID NO: 1).

In a further example according to the test kits and assay methods described herein, the bacteriophage polymerase is T3 RNA polymerase and the promoter sequence is a T3 RNA polymerase initiation sequence.

In a related example, the T3 RNA polymerase initiation sequence comprises a sequence defined by 5'-AATTAACCCTCACTAAAG-3' (SEQ ID NO: 2).

In yet a further example according to the test kits and assay methods described herein, the bacteriophage polymerase is SP6 RNA polymerase and the promoter sequence is a SP6 RNA polymerase initiation sequence.

In a related example, the SP6 RNA polymerase initiation sequence comprises a sequence defined by 5'-ATTTAGGTGACACTATAG-3' (SEQ ID NO: 3).

In yet another example according to the test kits and assay methods described herein, the reporter construct comprises a sequence encoding an RNA aptamer that when transcribed to form an RNA aptamer is capable of binding to a fluorophore thereby generating a fluorescence signal.

In a related example, the RNA aptamer is further supported by an RNA scaffold which promotes secondary structure formation of the RNA aptamer, thereby optimizing the binding interaction with its fluorophore(s). In a further related example, the RNA scaffold includes, but is not limited to, F30.

In another example, the RNA aptamer is Mango including, but not limited to, Mango I, Mango II, Mango III and Mango IV. In a related example, the fluorophore which binds to the Mango RNA aptamer thereby generating a fluorescent signal is a derivative of thiazole orange (TO).

In another example, the RNA aptamer is selected from Spinach, iSpinach, baby Spinach and Broccoli. In a related example, the fluorophore which binds to the Spinach or Broccoli RNA aptamer thereby generating a fluorescent signal is 3,5-difluoro-4-hydroxybenzylidene imidazolinone (DFHBI).

In another example, the RNA aptamer is Malachite Green. In a related example, the fluorophore which binds to the Malachite Green RNA aptamer thereby generating a flourescent signal is malachite green.

In a further example according to the test kits and assay methods described herein, the reporter construct comprises a single sequence copy of the RNA aptamer, or multiple sequence copies of the RNA aptamer. The term "multiple sequence copies" is intended to mean, without limitation, two, three, four, five, six, seven, eight, nine, 10, 11, 12, 13, 14, 15, 16, 17, 18, 19, 20, 21, 22, 23, 24, 25, 26, 27, 28, 29, 30, 31, 32 or more copies of the sequence encoding the RNA aptamer. A person skilled in the art will recognize that the copy number of RNA aptamer sequences will be governed by the optimal signal to noise ratio, as determined by routine assay optimization.

In other examples according to the test kits and assay methods described herein, the reporter construct is selected from the group consisting of a gene that does or does not encode a protein or polypeptide that can be detected by RNA analysis.

As such, the test kits and assay methods described herein may be configured to detect transcript levels of a reporter construct by investigating, for example, messenger ribonucleic acid (mRNA) or complementary deoxyribose nucleic acid (cDNA) levels when a test sample is combined with the assay, as a means to screen the sample for the presence of a ligand having steroid hormone receptor activity.

In yet a further example according to the test kits and assay methods described herein, a reduction or inhibition in transcription of a reporter construct may be measured using polymerase chain reaction (PCR) quantitative PCR (also known as real-time PCR, qPCR), digital PCR (dPCR), reverse transcription PCR (RT-PCR), reverse transcription qPCR (RTqPCR), reverse transcription digital PCR (RTdPCR), RNA seq or *insitu* hybridisation.

In other examples according to the test kits and assay methods described herein, reporter gene transcript levels comprising (e.g.) mRNA or cDNA may be semi-/quantified using established techniques such as quantitative polymerase chain reaction (qPCR, including RealTime qPCR and Reverse Transcription-qPCR), or using other techniques such as fluorescence based on detection of intercalating dyes or on direct addition of fluorescent nucleotides. For example, the use of fluorophores that bind to RNA aptamers to form RNA-fluorophore complexes, as described herein. These and other techniques would be known to a person skilled in the art.

The test kits and assay methods described herein are configured for detection of various ligands of both known and unknown structure which will bind to a steroid hormone receptor including androgen receptor (AR), estrogen receptor alpha (ER-α), estrogen receptor beta (ER-β), progesterone receptor A (PRA), progesterone receptor B (PRB), mineralocorticoid receptor (MR) and glucocorticoid receptor (GR).

Examples of ligands known to bind androgen receptor include, without limitation, Testosterone, Dihydrotestosterone; Androgenic Anabolic Steroids (AAS) including but not limited to TRENA, 17α-Trenbolone, 17β-Trenbolone, Trendione, Nandrolone, Boldenone, Altrenogest; Selective Adrogen Receptor Modulators (SARMs) including but not limited to 93746, BMS-546929, LGD4033, ACP105, YK-11, Andarine, Ligandrol, Ostarine.

Examples of ligands known to bind estrogen receptor alpha include, without limitation, Estradiol, Estrone, Estriol; Selective Estrogen Receptor Modulators including Raloxifene, Tamoxifen, Toremifene, Ospemifene, Lasofoxifene, Cyclofenil, Clomifene, Broparestrol, Basedoxifene, Anordrin; Phytoestrogens including but not limited to, dietary estrogens such as Polyphenols (Resveratrol), Flavanones (Eriodictyol, Hesperetin, Homoeriodictyol, Naringenin), Flavones (Apigenin, Luteolin, Tangeritin), Flavonols (Fisetin, Kaempferol, Myricetin, Pachypodol, Quercetin, Rhamnazin), Catechins (Proanthocyanides), Isoflavonoids (Isoflavones Biochanin A, Clycitein, Daidzein, Formononetin, Genistein), Isoflavans (Equol), Coumestans (Coumestrol); Estrogen-like Endocrine Disruptive Chemicals (EEDC) including, but not limited to, Dichlorodiphenyltrichloroethane (DDT), Dioxin, Polychlorinated Biphenyls (PCBs), Bisphenol A (BPA), Polybrominated Biphenyls (PBB), Phthalate Esters, Endosulfan, Atrazine, Zeranol; designer compounds such as Hydrazide Derivatives.

Examples of ligands known to bind estrogen receptor beta include, without limitation, all ligands which bind to estrogen receptor alpha, as well as, Diarylpropionitrile (DPN) and Wyeth-derived Benzoxazoles such as Way-659, Way-818 and Way-200070.

Examples of ligands known to bind progesterone receptor A and progesterone receptor B include, without limitation, Progesterone, Norethisterone, Levonorgestrel, Medroxyprogesterone Acetate, Megestrol Acetate, Dydrogesterone, Drospirenone; Selective Progesterone Receptor Modulators including Ulipristal Acetate, Telapristone Acetate, Vilaprisan, Asoprisnil, Asoprisnil Ecamate; Anti-Progestins including Mifepristone, Onapristone, Lilopritone and Gestrinone.

Examples of ligands known to bind mineralocorticoid receptor include, without limitation, Aldosterone; synthetic mineralocorticoids such as Fludrocortisone; antimineralocorticoids such as Spironolactone and Eplerenone; glucocorticoid receptor ligands such as those described below.

Examples of ligands known to bind glucocorticoid receptor include, without limitation dexamethasone, hydrocortisone, cortisone, prednisolone, methylprednisolone, prednisone, amcinonide, budesonide, desonide, fluocinonide, halcinonide, beclometasone, betamethasone, fluocortolone, halometasone, mometasone, or as antagonists mifepristone, and ketoconazole.

### Exemplary Assay Components & Constructs

Steroid hormone receptors activated by a ligand present in a sample will dimerize (i.e. forming a receptor-ligand complex as defined) and bind to its complimentary hormone response element. In the test kits and assays described herein, hormone response elements are linked to a reporter construct, and a change in a physical property of the reporter construct may be used to reflect the presence of a ligand in a sample under investigation. Exemplary hormone response elements according to the present invention include: androgen response element (ARE), estrogen response element (ERE), progesterone response element (PRE), mineralocorticoid response element (MRE) and glucocorticoid response element (GRE).

As previously stated, the various hormone response elements incorporate binding motifs configured to selectively bind activated ligand-receptor complexes. For example, each of the androgen, estrogen, progesterone, mineralocorticoid and glucocorticoid response elements comprise imperfect dihexameric palindrome sequences which in their secondary structure orientations facilitate binding of a dimerized ligand receptor complex (i.e. (HR-L)₂) via zinc finger binding motifs.

In an example according to the test kits and assay methods described herein, the androgen response element comprises a DNA binding motif that binds to an activated androgen receptor. In a related example, the DNA binding motif binds to a dimer of the ligand bound androgen receptor (i.e. (AR-L)₂; where "AR" is an androgen receptor and "L" is a ligand). In a related example, the DNA binding motif comprises imperfect dihexameric palindrome sequences which create binding specificity between the activated androgen receptor and an androgen response element. In a further related example, the androgen response element comprising a DNA binding motif is a double stranded deoxyribonucleic acid.

In an example according to the test kits and assay methods described herein, the estrogen response element comprises a DNA binding motif that binds to an activated estrogen receptor. In a related example, the DNA binding motif binds to a dimer of the ligand bound estrogen receptor (i.e. (ER-L)₂; where "ER" is an estrogen receptor selected from ER-α or ER-β). In a further related example, the DNA binding motif comprises imperfect dihexameric palindrome sequences which create binding specificity between the activated estrogen receptor and an estrogen response element. In a further related example, the estrogen response element comprising a DNA binding motif is a double stranded deoxyribonucleic acid.

In an example according to the test kits and assay methods described herein, the progesterone response element comprises a DNA binding motif that binds to an activated progesterone receptor. In a related example, the DNA binding motif binds to a dimer of the ligand bound progesterone receptor (i.e. (PR-L)₂; where "PR" is a progesterone receptor selected from PRA or PRB). In a further related example, the DNA binding motif comprises imperfect dihexameric palindrome sequences which create binding specificity between the activated progesterone receptor and a progesterone response element. In a further related example, the progesterone response element comprising a DNA binding motif is a double stranded deoxyribonucleic acid.

In an example according to the test kits and assay methods described herein, the mineralocorticoid response element comprises a DNA binding motif that selectively binds to an activated mineralocorticoid receptor. In a related example, the DNA binding motif binds to a dimer of the ligand bound mineralocorticoid receptor (i.e. (MR-L)₂; where "MR" is a mineralocorticoid receptor). In a further related example, the DNA binding motif comprises imperfect dihexameric palindrome sequences which create binding specificity between the activated mineralocorticoid receptor and a mineralocorticoid response element. In a further related example, the mineralocorticoid response element comprising a DNA binding motif is a double stranded deoxyribonucleic acid.

In an example according to the test kits and assay methods described herein, the glucocorticoid response element comprises a DNA binding motif that selectively binds to an activated glucocorticoid receptor. In a related example, the DNA binding motif binds to a dimer of the ligand bound glucocorticoid receptor (i.e. (GR-L)₂; where "GR" is a glucocorticoid receptor). In a further related example, the DNA binding motif comprises imperfect dihexameric palindrome sequences which create binding specificity between the activated glucocorticoid receptor and a glucocorticoid response element. In a further related example, the glucocorticoid response element comprising a DNA binding motif is a double stranded deoxyribonucleic acid.

Detection of a ligand that binds to and activates an androgen receptor, such as Testosterone, Dihydrotestosterone, synthetic steroid hormones (eg. AAS) and selective androgen receptor modulators (i.e SARMs) and phyto- or xenoandrogens, requires test kits/assays comprising an androgen receptor together with an androgen response element capable of binding to an activated androgen-receptor complex.

In an example according to the test kits and assay methods described herein, the androgen response element comprises or consist in the sequence 5'-AGAACAnnnTGTTCT-3' (SEQ ID NO: 4), where n is any nucleic acid base selected from G, C, T or A. Its complimentary antisense sequence is defined as 5'-AGAACAnnnTGTTCT-3' (SEQ ID NO: 5), where n represents the base that is complementary to SEQ ID NO: 4 based on a sequence alignment between SEQ ID NOs: 4 and 5 (i.e. A = T; T = A; G = C; C = G).

In another example according to the test kits and assay methods described herein, the androgen response element comprises or consist in the sequence 5'-GGTACAnnnTGTTCT-3' (SEQ ID NO: 6), where n is any nucleic acid base selected from G, C, T or A. Its complimentary antisense sequence is defined as 5'-AGAACAnnnTGTACC-3' (SEQ ID NO: 7), where n represents the base that is complementary to SEQ ID NO: 6 based on a sequence alignment between SEQ ID NOs: 6 and 7 (i.e. A = T; T = A; G = C; C = G).

Detection of a ligand that binds to and activates an estrogen receptor, such as Estradiol, Estrone, other estrogen-like steroid hormones including phyto- and xenoestrogens and selective estrogen receptor modulators (SERMs), requires test kits/assays comprising either an estrogen receptor alpha (ER-α) or estrogen receptor beta (ER-β) together with an estrogen response element capable of binding to an activated estrogen-receptor complex.

In an example according to the test kits and assay methods described herein, the estrogen response element comprises or consist in the sequence 5'-AGGTCAnnnTGACCT-3' (SEQ ID NO: 8), where n is any nucleic acid base selected from G, C, T or A. Its complimentary antisense sequence is defined as 5'-AGGTCAnnnTGACCT-3' (SEQ ID NO: 9), where n represents the base that is complementary to SEQ ID NO: 8 based on a sequence alignment between SEQ ID NOs: 8 and 9 (i.e. A = T; T = A; G = C; C = G).

Detection of a ligand that binds to and activates a progesterone receptor, such as Progesterone, Norethisterone, Levonorgestrel, other progesterone-like steroid hormones and selective progesterone receptor modulators (SPRMs), requires test kits/assays comprising either an progesterone receptor A (PRA) or progesterone receptor B (PRB) together with an progesterone response element capable of binding to an activated progesterone-receptor complex.

In an example according to the test kits and assay methods described herein, the progesterone response element comprises or consist in the sequence 5'-GGTACAAACTGTTCT-3' (SEQ ID NO: 10). Its complimentary antisense sequence is defined as 5'-AGAACAGTTTGTACC-3' (SEQ ID NO: 11).

Detection of a ligand that binds to and activates a mineralocorticoid receptor, such as Aldosterone, synthetic mineralocorticoids such as Fludrocortisone and antimineralocorticoids such as Spironolactone and Eplerenone, requires test kits/assays comprising a mineralocorticoid receptor together with an mineralocorticoid response element capable of binding to an activated mineralocorticoid-receptor complex.

In an example according to the test kits and assay methods described herein, the mineralocorticoid response element comprises or consist in the sequence 5'-AGAACAnAATGTTCT-3' (SEQ ID NO: 12), where n is any nucleic acid base selected from G, C, T or A. Its complimentary antisense sequence is defined as 5'-AGAACATTnTGTTCT-3' (SEQ ID NO: 13), where n represents the base that is complementary to SEQ ID NO: 12 based on a sequence alignment between SEQ ID NOs: 12 and 13 (i.e. A = T; T = A; G = C; C = G).

Detection of a ligand that binds to and activates a glucocorticoid receptor, such as, Cortisol, Dexamethasone and 11-Dihydrocorticosterone requires test kits/assays comprising a glucocorticoid receptor together with an glucocorticoid response element capable of binding to an activated glucocorticoid-receptor complex.

In an example according to the test kits and assay methods described herein, the glucocorticoid response element comprises or consist in the sequence 5'-AGAACAnAATGTTCT-3' (SEQ ID NO: 12), where n is any nucleic acid base selected from G, C, T or A. Its complimentary antisense sequence is defined as 5'-AGAACATTnTGTTCT-3' (SEQ ID NO: 13), where n represents the base that is complementary to SEQ ID NO: 12 based on a sequence alignment between SEQ ID NOs: 12 and 13 (i.e. A = T; T = A; G = C; C = G).

Exemplary nucleic acid sequences according to the test kits and assay methods described here include, without limitation:
T7i-ARE(n)-MangoII: where n=1,2,3,4;
T7i-ERE(n)-MangoII: where n=1,2,3,4;
T7i-PRE(n)-MangoII: where n=1,2,3,4;
T7i-MRE(n)-MangoII: where n=1,2,3,4;
T7i-GRE(n)-MangoII: where n=1,2,3,4;
T7i-ARE(n)-F30-MangoII: where n=1,2,3,4;
T7i-ERE(n)-F30-MangoII: where n=1,2,3,4;
T7i-PRE(n)-F30-MangoII: where n=1,2,3,4;
T7i-MRE(n)-F30-MangoII: where n=1,2,3,4;
T7i-GRE(n)-F30-MangoII: where n=1,2,3,4;
T7i-ARE(n)-iSpinach: where n=1,2,3,4;
T7i-ERE(n)-iSpinach: where n=1,2,3,4;
T7i-PRE(n)-iSpinach: where n=1,2,3,4;
T7i-MRE(n)-iSpinach: where n=1,2,3,4;
T7i-GRE(n)-iSpinach: where n=1,2,3,4;
T7i-ARE(n)-F30-iSpinach: where n=1,2,3,4;
T7i-ERE(n)-F30-iSpinach: where n=1,2,3,4;
T7i-PRE(n)-F30-iSpinach: where n=1,2,3,4;
T7i-MRE(n)-F30-iSpinach: where n=1,2,3,4;
T7i-GRE(n)-F30-iSpinach: where n=1,2,3,4; where T7i represents a promoter/initiation sequence for T7 RNA polymerase.

In a related example, the T7i promoter/initiation sequence is defined by 5'-TAATACGACTCACTATAG-3' (SEQ ID NO: 1).

Alternate exemplary nucleic acid sequences according to the test kits and assay methods described here include, without limitation:
T3i-ARE(n)-MangoII: where n=1,2,3,4;
T3i-ERE(n)-MangoII: where n=1,2,3,4;
T3i-PRE(n)-MangoII: where n=1,2,3,4;
T3i-MRE(n)-MangoII: where n=1,2,3,4;
T3i-GRE(n)-MangoII: where n=1,2,3,4;
T3i-ARE(n)-F30-MangoII: where n=1,2,3,4;
T3i-ERE(n)-F30-MangoII: where n=1,2,3,4;
T3i-PRE(n)-F30-MangoII: where n=1,2,3,4;
T3i-MRE(n)-F30-MangoII: where n=1,2,3,4;
T3i-GRE(n)-F30-MangoII: where n=1,2,3,4;
T3i-ARE(n)-iSpinach: where n=1,2,3,4;
T3i-ERE(n)-iSpinach: where n=1,2,3,4;
T3i-PRE(n)-iSpinach: where n=1,2,3,4;
T3i-MRE(n)-iSpinach: where n=1,2,3,4;
T3i-GRE(n)-iSpinach: where n=1,2,3,4;
T3i-ARE(n)-iSpinachx3: where n=1,2,3,4;
T3i-ERE(n)-iSpinachx3: where n=1,2,3,4;
T3i-PRE(n)-iSpinachx3: where n=1,2,3,4;
T3i-MRE(n)-iSpinachx3: where n=1,2,3,4;
T3i-GRE(n)-iSpinachx3: where n=1,2,3,4;
where T3i represents a promoter or initiation sequence for T3 RNA polymerase.

In a related example, the T3i promoter/initiation sequence is defined by 5'-AATTAACCCTCACTAAAG-3' (SEQ ID NO: 2).

Alternate exemplary nucleic acid sequences according to the test kits and assay methods described here include, without limitation:
SP6i-ARE(n)-MangoII: where n=1,2,3,4;
SP6i-ERE(n)-MangoII: where n=1,2,3,4;
SP6i-PRE(n)-MangoII: where n=1,2,3,4;
SP6i-MRE(n)-MangoII: where n=1,2,3,4;
SP6i-GRE(n)-MangoII: where n=1,2,3,4;
SP6i-ARE(n)-F30-MangoII: where n=1,2,3,4;
SP6i-ERE(n)-F30-MangoII: where n=1,2,3,4;
SP6i-PRE(n)-F30-MangoII: where n=1,2,3,4;
SP6i-MRE(n)-F30-MangoII: where n=1,2,3,4;
SP6i-GRE(n)-F30-MangoII: where n=1,2,3,4;
SP6i-ARE(n)-iSpinach: where n=1,2,3,4;
SP6i-ERE(n)-iSpinach: where n=1,2,3,4;
SP6i-PRE(n)-iSpinach: where n=1,2,3,4;
SP6i-MRE(n)-iSpinach: where n=1,2,3,4;
SP6i-GRE(n)-iSpinach: where n=1,2,3,4;
SP6i-ARE(n)-iSpinachx3: where n=1,2,3,4;
SP6i-ERE(n)-iSpinachx3: where n=1,2,3,4;
SP6i-PRE(n)-iSpinachx3: where n=1,2,3,4;
SP6i-MRE(n)-iSpinachx3: where n=1,2,3,4;
SP6i-GRE(n)-iSpinachx3: where n=1,2,3,4;
where SP6i represents a promoter or initiation sequence for SP6 RNA polymerase.

In a related example, the SP6i promoter/initiation sequence is defined by 5'-ATTTAGGTGACACTATAG-3' (SEQ ID NO: 3).

In another example according to the present invention, the test kits and/or assay methods are configured to detect ligands that bind to an androgen receptor, and the nucleic acid sequence comprised of a T7 RNA polymerase initiation sequence, an androgen response element and a reporter construct encoding F30 scaffold and Mango II RNA aptamer comprises the sequence set forth in SEQ ID NO: 14 as follows:

[SEQ ID NO: 14]
5′TAATACGACTCACTATAGACTCTGGAGGAATGGAGAACAGCCTGTTCTCCATTGCCATGTGTATGT
GGGTACGAAGGAGAGGAGAGGAAGAGGAGAGTACCCACATACTCTGATGATCCTTCGGGATCATTC
ATGGCAATCTAGGA3′

In yet another example according to the present invention, the test kits and/or assay methods are configured to detect ligands that bind to an estrogen receptor alpha or estrogen receptor beta, and the nucleic acid sequence comprised of a T7 RNA polymerase initiation sequence, an estrogen response element and a reporter construct encoding F30 scaffold and Mango II RNA aptamer comprises the sequence set forth in SEQ ID NO: 15 as follows:

[SEQ ID NO: 15]
5'TAATACGACTCACTATAGACTCTGGAGGAACAGGTCAGCATGACCTGTTGCCATGTGTATGTGGGT
ACGAAGGAGAGGAGAGGAAGAGGAGAGTACCCACATACTCTGATGATCCTTCGGGATCATTCATGG
CAATCTAGGA3'

In yet a further another example according to the present invention, the test kits and/or assay methods are configured to detect ligands that bind to a progesterone receptor A or a progesterone receptor B, and the nucleic acid sequence comprised of a T7 RNA polymerase initiation sequence, an progesterone response element and a reporter construct encoding F30 scaffold and Mango II RNA aptamer comprises the sequence set forth in SEQ ID NO: 16 as follows:

[SEQ ID NO: 16]
5'TAATACGACTCACTATAGACTCTGGAGGAAGGTACAAACTGTTCTTTGCCATGTGTATGTGGGTAC
GAAGGAGAGGAGAGGAAGAGGAGAGTACCCACATACTCTGATGATCCTTCGGGATCATTCATGGCA
ATCTAGGA3'

In another example according to the present invention, the test kits and/or assay methods are configured to detect ligands that bind to a mineralocorticoid receptor, and the nucleic acid sequence comprised of a T7 RNA polymerase initiation sequence, an mineralocorticoid response element and a reporter construct encoding F30 scaffold and Mango II RNA aptamer comprises the sequence set forth in SEQ ID NO: 17 as follows:

[SEQ ID NO: 17]
5'TAATACGACTCACTATAGACTCTGGAGGAATGTACAGGATGTTCTTTGCCATGTGTATGTGGGTAC
GAAGGAGAGGAGAGGAAGAGGAGAGTACCCACATACTCTGATGATCCTTCGGGATCATTCATGGCA
ATCTAGGA3'

In another example according to the present invention, the test kits and/or assay methods are configured to detect ligands that bind to a glucocorticoid receptor, and the nucleic acid sequence comprised of a T7 RNA polymerase initiation sequence, an glucocorticoid response element and a reporter construct encoding F30 scaffold and Mango II RNA aptamer comprises the sequence set forth in SEQ ID NO: 18 as follows:

[SEQ ID NO: 18]
5'TAATACGACTCACTATAGACTCTGGAGGAATGTACAGGATGTTCTTTGCCATGTGTATGTGGGTAC
GAAGGAGAGGAGAGGAAGAGGAGAGTACCCACATACTCTGATGATCCTTCGGGATCATTCATGGCA
ATCTAGGA3'

Other nucleic acids/constructs for use in the test kits and assay methods according to the present invention are summarised in Table 1, below.

**Table 1: Exemplary Construct Sequence Information**

| **CONSTRUCT** | **SEQUENCE** | **SEQUENCE ID NO:** |
|---|---|---|
| T7i-ARE-MangoII | TAATACGACTCACTATAGACTCTGGAGGAATGGAGAACAGCCTGTTCT CCATACGAAGGAGAGGAGAGGAAGAGGAGAGTA | 19 |
| T7i-ERE-MangoII | TAATACGACTCACTATAGACTCTGGAGGAACAGGTCAGCATGACCTGT ACGAAGGAGAGGAGAGGAAGAGGAGAGTA | 20 |
| T7i-PRE-MangoII | TAATACGACTCACTATAGACTCTGGAGGAAGGTACAAACTGTTCTTAC GAAGGAGAGGAGAGGAAGAGGAGAGTA | 21 |
| T7i-MRE-MangoII | TAATACGACTCACTATAGACTCTGGAGGAATGTACAGGATGTTCTTAC GAAGGAGAGGAGAGGAAGAGGAGAGTA | 22 |
| T7i-GRE-MangoII | TAATACGACTCACTATAGACTCTGGAGGAATGTACAGGATGTTCTTAC GAAGGAGAGGAGAGGAAGAGGAGAGTA | 23 |
| T7i-ARE-iSpinach | TAATACGACTCACTATAGACTCTGGAGGAATGGAGAACAGCCTGTTCT CCAAGGAGTACGGTGAGGGTCGGGTCCAGTAGGTACGCCTACTGTTG AGTAGAGTGTGGGCTCCGTACTCCC | 24 |
| T7i-ERE-iSpinach | TAATACGACTCACTATAGACTCTGGAGGAACAGGTCAGCATGACCTGA GGAGTACGGTGAGGGTCGGGTCCAGTAGGTACGCCTACTGTTGAGTA GAGTGTGGGCTCCGTACTCCC | 25 |
| T7i-PRE-iSpinach | TAATACGACTCACTATAGACTCTGGAGGAAGGTACAAACTGTTCTAGG AGTACGGTGAGGGTCGGGTCCAGTAGGTACGCCTACTGTTGAGTAGA GTGTGGGCTCCGTACTCCC | 26 |
| T7i-MRE-iSpinach | TAATACGACTCACTATAGACTCTGGAGGAATGTACAGGATGTTCTAGG AGTACGGTGAGGGTCGGGTCCAGTAGGTACGCCTACTGTTGAGTAGA GTGTGGGCTCCGTACTCCC | 27 |
| T7i-GRE-iSpinach | TAATACGACTCACTATAGACTCTGGAGGAATGTACAGGATGTTCTAGG AGTACGGTGAGGGTCGGGTCCAGTAGGTACGCCTACTGTTGAGTAGA GTGTGGGCTCCGTACTCCC | 28 |
| T7i-ARE-F30iSpinach | TAATACGACTCACTATAGACTCTGGAGGAATGGAGAACAGCCTGTTCT CCATTGCCATGTGTATGTGGGAGGAGTACGGTGAGGGTCGGGTCCAG TAGGTACGCCTACTGTTGAGTAGAGTGTGGGCTCCGTACTCCCCCCAC ATACTCTGATGATCCTTCGGGATCATTCATGGCAATCTAGATCTAGA | 29 |
| T7i-ERE-F30iSpinach | TAATACGACTCACTATAGACTCTGGAGGAACAGGTCAGCATGACCTGT TGCCATGTGTATGTGGGAGGAGTACGGTGAGGGTCGGGTCCAGTAG GTACGCCTACTGTTGAGTAGAGTGTGGGCTCCGTACTCCCCCCACATA CTCTGATGATCCTTCGGGATCATTCATGGCAATCTAGATCTAGA | 30 |
| T7i-PRE-F30iSpinach | TAATACGACTCACTATAGACTCTGGAGGAAGGTACAAACTGTTCTTTG CCATGTGTATGTGGGAGGAGTACGGTGAGGGTCGGGTCCAGTAGGTA CGCCTACTGTTGAGTAGAGTGTGGGCTCCGTACTCCCCCCACATACTC TGATGATCCTTCGGGATCATTCATGGCAATCTAGATCTAGA | 31 |
| T7i-MRE-F30iSpinach | TAATACGACTCACTATAGACTCTGGAGGAATGTACAGGATGTTCTTTG CCATGTGTATGTGGGAGGAGTACGGTGAGGGTCGGGTCCAGTAGGTA CGCCTACTGTTGAGTAGAGTGTGGGCTCCGTACTCCCCCCACATACTC TGATGATCCTTCGGGATCATTCATGGCAATCTAGATCTAGA | 32 |
| T7i-GRE-F30iSpinach | TAATACGACTCACTATAGACTCTGGAGGAATGTACAGGATGTTCTTTG CCATGTGTATGTGGGAGGAGTACGGTGAGGGTCGGGTCCAGTAGGTA CGCCTACTGTTGAGTAGAGTGTGGGCTCCGTACTCCCCCCACATACTC TGATGATCCTTCGGGATCATTCATGGCAATCTAGATCTAGA | 33 |
| T3i-ARE-MangoII | AATTAACCCTCACTAAAGACTCTGGAGGAATGGAGAACAGCCTGTTCT CCATACGAAGGAGAGGAGAGGAAGAGGAGAGTA | 34 |
| T3i-ERE-MangoII | AATTAACCCTCACTAAAGACTCTGGAGGAACAGGTCAGCATGACCTGT ACGAAGGAGAGGAGAGGAAGAGGAGAGTA | 35 |
| T3i-PRE-MangoII | AATTAACCCTCACTAAAGACTCTGGAGGAAGGTACAAACTGTTCTTAC GAAGGAGAGGAGAGGAAGAGGAGAGTA | 36 |
| T3i-MRE-MangoII | AATTAACCCTCACTAAAGACTCTGGAGGAATGTACAGGATGTTCTTAC GAAGGAGAGGAGAGGAAGAGGAGAGTA | 37 |
| T3i-GRE-MangoII | AATTAACCCTCACTAAAGACTCTGGAGGAATGTACAGGATGTTCTTAC GAAGGAGAGGAGAGGAAGAGGAGAGTA | 38 |
| T3i-ARE-F30MangoII | AATTAACCCTCACTAAAGACTCTGGAGGAATGGAGAACAGCCTGTTCT CCATTGCCATGTGTATGTGGGTACGAAGGAGAGGAGAGGAAGAGGA GAGTACCCACATACTCTGATGATCCTTCGGGATCATTCATGGCAATCT AGGA | 39 |
| T3i-ERE-F30MangoII | AATTAACCCTCACTAAAGACTCTGGAGGAACAGGTCAGCATGACCTG TTGCCATGTGTATGTGGGTACGAAGGAGAGGAGAGGAAGAGGAGAG TACCCACATACTCTGATGATCCTTCGGGATCATTCATGGCAATCTAGG A | 40 |
| T3i-PRE-F30MangoII | AATTAACCCTCACTAAAGACTCTGGAGGAAGGTACAAACTGTTCT TTGCCATGTGTATGTGGGTACGAAGGAGAGGAGAGGAAGAGGAGAG TACCCACATACTCTGATGATCCTTCGGGATCATTCATGGCAATCTAGG A | 41 |
| T3i-MRE-F30MangoII | AATTAACCCTCACTAAAGACTCTGGAGGAATGTACAGGATGTTCT TTGCCATGTGTATGTGGGTACGAAGGAGAGGAGAGGAAGAGGAGAG TACCCACATACTCTGATGATCCTTCGGGATCATTCATGGCAATCTAGG A | 42 |
| T3i-GRE-F30MangoII | AATTAACCCTCACTAAAGACTCTGGAGGAATGTACAGGATGTTCT TTGCCATGTGTATGTGGGTACGAAGGAGAGGAGAGGAAGAGGAGAG TACCCACATACTCTGATGATCCTTCGGGATCATTCATGGCAATCTAGG A | 43 |
| T3i-ARE-iSpinach | AATTAACCCTCACTAAAGACTCTGGAGGAATGGAGAACAGCCTGTTCT CCAAGGAGTACGGTGAGGGTCGGGTCCAGTAGGTACGCCTACTGTTG AGTAGAGTGTGGGCTCCGTACTCCC | 44 |
| T3i-ERE-iSpinach | AATTAACCCTCACTAAAGACTCTGGAGGAACAGGTCAGCATGACCTGA GGAGTACGGTGAGGGTCGGGTCCAGTAGGTACGCCTACTGTTGAGTA GAGTGTGGGCTCCGTACTCCC | 45 |
| T3i-PRE-iSpinach | AATTAACCCTCACTAAAGACTCTGGAGGAAGGTACAAACTGTTCTAGG AGTACGGTGAGGGTCGGGTCCAGTAGGTACGCCTACTGTTGAGTAGA GTGTGGGCTCCGTACTCCC | 46 |
| T3i-MRE-iSpinach | AATTAACCCTCACTAAAGACTCTGGAGGAATGTACAGGATGTTCTAGG AGTACGGTGAGGGTCGGGTCCAGTAGGTACGCCTACTGTTGAGTAGA GTGTGGGCTCCGTACTCCC | 47 |
| T3i-GRE-iSpinach | AATTAACCCTCACTAAAGACTCTGGAGGAATGTACAGGATGTTCTAGG AGTACGGTGAGGGTCGGGTCCAGTAGGTACGCCTACTGTTGAGTAGA GTGTGGGCTCCGTACTCCC | 48 |
| T3i-ARE-F30iSpinach | AATTAACCCTCACTAAAGACTCTGGAGGAATGGAGAACAGCCTGTTCT CCATTGCCATGTGTATGTGGGAGGAGTACGGTGAGGGTCGGGTCCAG TAGGTACGCCTACTGTTGAGTAGAGTGTGGGCTCCGTACTCCCCCCAC ATACTCTGATGATCCTTCGGGATCATTCATGGCAATCTAGATCTAGA | 49 |
| T3i-ERE-F30iSpinach | AATTAACCCTCACTAAAGACTCTGGAGGAACAGGTCAGCATGACCTGT TGCCATGTGTATGTGGGAGGAGTACGGTGAGGGTCGGGTCCAGTAG GTACGCCTACTGTTGAGTAGAGTGTGGGCTCCGTACTCCCCCCACATA CTCTGATGATCCTTCGGGATCATTCATGGCAATCTAGATCTAGA | 50 |
| T3i-PRE-F30iSpinach | AATTAACCCTCACTAAAGACTCTGGAGGAAGGTACAAACTGTTCTTTG CCATGTGTATGTGGGAGGAGTACGGTGAGGGTCGGGTCCAGTAGGTA CGCCTACTGTTGAGTAGAGTGTGGGCTCCGTACTCCCCCCACATACTC TGATGATCCTTCGGGATCATTCATGGCAATCTAGATCTAGA | 51 |
| T3i-MRE-F30iSpinach | AATTAACCCTCACTAAAGACTCTGGAGGAATGTACAGGATGTTCTTTG CCATGTGTATGTGGGAGGAGTACGGTGAGGGTCGGGTCCAGTAGGTA CGCCTACTGTTGAGTAGAGTGTGGGCTCCGTACTCCCCCCACATACTC TGATGATCCTTCGGGATCATTCATGGCAATCTAGATCTAGA | 52 |
| T3i-GRE-F30iSpinach | AATTAACCCTCACTAAAGACTCTGGAGGAATGTACAGGATGTTCTTTG CCATGTGTATGTGGGAGGAGTACGGTGAGGGTCGGGTCCAGTAGGTA CGCCTACTGTTGAGTAGAGTGTGGGCTCCGTACTCCCCCCACATACTC TGATGATCCTTCGGGATCATTCATGGCAATCTAGATCTAGA | 53 |
| SP6i-ARE-MangoII | ATTTAGGTGACACTATAGACTCTGGAGGAATGGAGAACAGCCTGTTCT CCATACGAAGGAGAGGAGAGGAAGAGGAGAGTA | 54 |
| SP6i-ERE-MangoII | ATTTAGGTGACACTATAGACTCTGGAGGAACAGGTCAGCATGACCTGT ACGAAGGAGAGGAGAGGAAGAGGAGAGTA | 55 |
| SP6i-PRE-MangoII | ATTTAGGTGACACTATAGACTCTGGAGGAAGGTACAAACTGTTCTTAC GAAGGAGAGGAGAGGAAGAGGAGAGTA | 56 |
| SP6i-MRE-MangoII | ATTTAGGTGACACTATAGACTCTGGAGGAATGTACAGGATGTTCTTAC GAAGGAGAGGAGAGGAAGAGGAGAGTA | 57 |
| SP6i-GRE-MangoII | ATTTAGGTGACACTATAGACTCTGGAGGAATGTACAGGATGTTCTTAC GAAGGAGAGGAGAGGAAGAGGAGAGTA | 58 |
| SP6i-ARE-F30MangoII | ATTTAGGTGACACTATAGACTCTGGAGGAATGGAGAACAGCCTGTTCT CCATTGCCATGTGTATGTGGGTACGAAGGAGAGGAGAGGAAGAGGA GAGTACCCACATACTCTGATGATCCTTCGGGATCATTCATGGCAATCT AGGA | 59 |
| SP6i-ERE-F30MangoII | ATTTAGGTGACACTATAGACTCTGGAGGAACAGGTCAGCATGACCTGT TGCCATGTGTATGTGGGTACGAAGGAGAGGAGAGGAAGAGGAGAGT ACCCACATACTCTGATGATCCTTCGGGATCATTCATGGCAATCTAGGA | 60 |
| SP6i-PRE-F30MangoII | ATTTAGGTGACACTATAGACTCTGGAGGAAGGTACAAACTGTTCTTTG CCATGTGTATGTGGGTACGAAGGAGAGGAGAGGAAGAGGAGAGTAC CCACATACTCTGATGATCCTTCGGGATCATTCATGGCAATCTAGGA | 61 |
| SP6i-MRE-F30MangoII | ATTTAGGTGACACTATAGACTCTGGAGGAATGTACAGGATGTTCTTTG CCATGTGTATGTGGGTACGAAGGAGAGGAGAGGAAGAGGAGAGTAC CCACATACTCTGATGATCCTTCGGGATCATTCATGGCAATCTAGGA | 62 |
| SP6i-GRE-F30MangoII | ATTTAGGTGACACTATAGACTCTGGAGGAATGTACAGGATGTTCTTTG CCATGTGTATGTGGGTACGAAGGAGAGGAGAGGAAGAGGAGAGTAC CCACATACTCTGATGATCCTTCGGGATCATTCATGGCAATCTAGGA | 63 |
| SP6i-ARE-iSpinach | ATTTAGGTGACACTATAGACTCTGGAGGAATGGAGAACAGCCTGTTCT CCAAGGAGTACGGTGAGGGTCGGGTCCAGTAGGTACGCCTACTGTTG AGTAGAGTGTGGGCTCCGTACTCCC | 64 |
| SP6i-ERE-iSpinach | ATTTAGGTGACACTATAGACTCTGGAGGAACAGGTCAGCATGACCTGA GGAGTACGGTGAGGGTCGGGTCCAGTAGGTACGCCTACTGTTGAGTA GAGTGTGGGCTCCGTACTCCC | 65 |
| SP6i-PRE-iSpinach | ATTTAGGTGACACTATAGACTCTGGAGGAAGGTACAAACTGTTCTAGG AGTACGGTGAGGGTCGGGTCCAGTAGGTACGCCTACTGTTGAGTAGA GTGTGGGCTCCGTACTCCC | 66 |
| SP6i-MRE-iSpinach | ATTTAGGTGACACTATAGACTCTGGAGGAATGTACAGGATGTTCTAGG AGTACGGTGAGGGTCGGGTCCAGTAGGTACGCCTACTGTTGAGTAGA GTGTGGGCTCCGTACTCCC | 67 |
| SP6i-GRE-iSpinach | ATTTAGGTGACACTATAGACTCTGGAGGAATGTACAGGATGTTCTAGG AGTACGGTGAGGGTCGGGTCCAGTAGGTACGCCTACTGTTGAGTAGA GTGTGGGCTCCGTACTCCC | 68 |
| SP6i-ARE-F30iSpinach | ATTTAGGTGACACTATAGACTCTGGAGGAATGGAGAACAGCCTGTTCT CCATTGCCATGTGTATGTGGGAGGAGTACGGTGAGGGTCGGGTCCAG TAGGTACGCCTACTGTTGAGTAGAGTGTGGGCTCCGTACTCCCCCCAC ATACTCTGATGATCCTTCGGGATCATTCATGGCAATCTAGATCTAGA | 69 |
| SP6i-ERE-F30iSpinach | ATTTAGGTGACACTATAGACTCTGGAGGAACAGGTCAGCATGACCTGT TGCCATGTGTATGTGGGAGGAGTACGGTGAGGGTCGGGTCCAGTAG GTACGCCTACTGTTGAGTAGAGTGTGGGCTCCGTACTCCCCCCACATA CTCTGATGATCCTTCGGGATCATTCATGGCAATCTAGATCTAGA | 70 |
| SP6i-PRE-F30iSpinach | ATTTAGGTGACACTATAGACTCTGGAGGAAGGTACAAACTGTTCTTTG CCATGTGTATGTGGGAGGAGTACGGTGAGGGTCGGGTCCAGTAGGTA | 71 |
| | CGCCTACTGTTGAGTAGAGTGTGGGCTCCGTACTCCCCCCACATACTC TGATGATCCTTCGGGATCATTCATGGCAATCTAGATCTAGA | |
| SP6i-MRE-F30iSpinach | ATTTAGGTGACACTATAGACTCTGGAGGAATGTACAGGATGTTCTTTG CCATGTGTATGTGGGAGGAGTACGGTGAGGGTCGGGTCCAGTAGGTA CGCCTACTGTTGAGTAGAGTGTGGGCTCCGTACTCCCCCCACATACTC TGATGATCCTTCGGGATCATTCATGGCAATCTAGATCTAGA | 72 |
| SP6i-GRE-F30iSpinach | ATTTAGGTGACACTATAGACTCTGGAGGAATGTACAGGATGTTCTTTG CCATGTGTATGTGGGAGGAGTACGGTGAGGGTCGGGTCCAGTAGGTA CGCCTACTGTTGAGTAGAGTGTGGGCTCCGTACTCCCCCCACATACTC TGATGATCCTTCGGGATCATTCATGGCAATCTAGATCTAGA | 73 |

### Multiplexed Assay Systems

The present invention further contemplates multiplexed assays configured to detect two or more steroid hormone genomic responses from the *same* test sample.

To further illustrate the relevance of multiplexed systems, in certain circumstances it would be useful for a clinician investigating, for example, the hormonal status of a subject to know both the androgenic and estrogenic levels/activity in the subject.

The side-by-side detection of androgenic and estrogenic ligands from the same test sample is possible because a ligand which binds to an androgen receptor will not bind to and activate an estrogen receptor present in the same assay; conversely a ligand which binds to an estrogen receptor which will not bind to and activate an androgen receptor also present in the same assay. This is because androgen and estrogen receptors belong to different steroid hormone receptor classes, and so there is no 'cross-talk' in terms of receptor activation. And so, multiplexed assays have been developed to detect both androgenic and estrogenic ligands from the same sample.

Accordingly, in another aspect of the present invention there is provided a test kit for screening a sample for the side-by-side detection of an androgenic ligand and/or an estrogenic ligand, the test kit comprising:
(i) an androgen receptor, wherein the androgen receptor is capable of forming an androgen receptor-ligand complex with a complimentary ligand from the sample; and
(ii) a first nucleic acid molecule comprising:
   (a) a polymerase promoter sequence;
   (b) an androgen response element that is capable of being bound by the androgen receptor-ligand complex; and
   (c) a first reporter construct;
   where the response element (b) is located between the promoter sequence (a) and the reporter construct (c), and (a), (b) and (c) are operably linked; and
(iii) an estrogen receptor, wherein the estrogen receptor is capable of forming an estrogen receptor-ligand complex with a complimentary ligand from the sample; and
(iv) a second nucleic acid molecule comprising:
   (d) the polymerase promoter sequence;
   (e) an estrogen response element that is capable of being bound by the estrogen receptor-ligand complex; and
   (f) a second reporter construct; and
(iv) a single polypeptide polymerase

wherein, the first and second reporter constructs are different,
and wherein, the presence of an androgenic ligand in the sample is detected by measuring a reduction or inhibition in transcription of the first reporter construct caused by binding of the androgen receptor-ligand complex to the response element when the sample is combined with the test kit,
and wherein the presence of an estrogenic ligand in the sample is detected by measuring a reduction or inhibition in transcription of the second reporter construct caused by binding of the estrogen receptor-ligand complex to the response element when the sample is combined with the test kit.

In an example according to this aspect of the present invention, the test kit further comprises heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.

In an example according to this aspect of the present invention, the first and second nucleic acid molecules are discrete molecules.

In another example according to this aspect of the present invention, the first and second nucleic acid molecules are operably linked.

In yet another example according to this aspect of the present invention, the first nucleic acid molecule comprises a sequence defined by SEQ ID NO: 14.

In a further example according to this aspect of the present invention, the second nucleic acid molecule comprises a sequence defined by SEQ ID NO: 15.

In yet a further example according to this aspect of the present invention the first and second nucleic acid molecules are operably linked and comprise a sequence defined by SEQ ID NO: 80.

5′-
TAATACGACTCACTATAGACTCTGGAGGAATGGAGAACAGCCTGTTCTCCATTGCCATGTGTATGTG
GGTACGAAGGAGAGGAGAGGAAGAGGAGAGTACCCACATACTCTGATGATCCTTCGGGATCATTCA
TGGCAATCTAGGAAACCCCGCGGGGCCTTTCGGGGGTCTCGCGGGGTTTTTTGCTCTAATACGACT
CACTATAGACTCTGGAGGAACAGGTCAGCATGACCTGTTGCCATGTGTATGTGGGTACGAAGGAGA
GGAGAGGAAGAGGAGAGTACCCACATACTCTGATGATCCTTCGGGATCATTCATGGCAATCTAGGA-3′

Advantageously, the assays and test kits described herein are particularly suited for configuration in multiplexed systems because (i) their performance is possible in the absence of a cell-extract which otherwise contains naturally occuring ligands and/or steriod hormone receptors that interfere with the assay signal (e.g. ligand and receptor 'cross-talk' leads to autoactivation of the response element) and (ii) the simplicity of the assay systems described herein means that an existing assay or test kit may be routinely modified to include a second or subsequent receptor/report construct combination specific to detection of a second ligand; by leveraging the same *in vitro* transcription machinary (e.g. bacteriophage polymerase + nucleoside triphosphates), discrete signals generated by each reporter may be conveniently detected. To further illustrate this point, a multiplexed assay system according to the present invention may comprise, for example, an androgen specific reporter construct which, in the presence of androgen or an androgen-like ligand, would generate a reporter read-out that may be measured independently of the read-out generated by a reporter construct that is specific for the detection of estradiol in the same sample.

The skilled person would appreciate the advantages conferred by a lack of molecular complexity associated with the multiplexed systems of the present invention, and would recognise that detection of multiple *discrete* steroid hormone genomic responses (e.g. two, three, four, or more) from the same test sample is possible.

Accordingly, the test kits according to the present invention comprise at least one steriod hormone receptor and at least one nucleic acid molecule comprising at least one reporter construct.

Accordingly, the term "a steroid hormone receptor" according to the test kits and methods described herein is intended to mean "at least one steriod hormone receptor" in the sense that two or more *different types* of sterioid hormone receptors may be present (e.g. and by way of illustration only, a steroid hormone receptor that binds testosterone and a steroid hormone receptor that binds estradiol).

Similarly, the term "a nucleic acid molecule [comprising a response element]" is intended to mean "at least one nucleic acid" in the sense that two or more discrete nucleic acid molecules may be present, each comprising a different response element and optionally a different reporter molecule.

In an example according to this aspect of the present invention, the test kit comprises (i) an estrogen receptor and nucleic acid molecule comprising an estrogen response element, and (ii) an androgen receptor and nucleic acid molecule comprising an androgen response element.

In a related example, the nucleic acid molecule comprising an estrogen response element further comprises a first RNA aptamer that is capable of binding to a first fluorophore.

In another related example, the nucleic acid molecule comprising the androgen response element further comprises a second RNA aptamer that is capable of binding to a second fluorophore.

In a related example, the first and second RNA aptamers include, but are not limited to Mango I, Mango II, Mango III and Mango IV, Spinach, iSpinach, baby Spinach, Broccoli and Malachite Green, provided the first RNA aptamer is not identical to the second RNA aptamer.

In a further related example, the first RNA aptamer is Mango II and the second RNA aptamer is Malachite Green.

In a further related example, the first RNA aptamer is Mango II and the second RNA aptamer is iSpinach.

### Utility of the Test Kits & Assays

Advantageously, the present invention provides activity based test kits, assays and methods that work fundamentally on the principle of steroid hormone receptor activation. By detecting steroid hormone receptor activation (with consequent binding to its hormone response element) by a target ligand present within a sample to be tested, the present invention provides cell-free test kits, assays and methods that do not rely on structural knowledge of the ligand(s) being interrogated, can readily distinguish between the presence of biologically active and inactive ligands, and provide cost-effective, reliable and reproducible systems that do not require complex laboratory equipment or particular expertise to perform.

Accordingly, in another aspect of the present invention there is provided a method for determining the doping status of an athlete, the method comprising combining a sample obtained from the athlete with a test kit as described herein and determining the doping status of an athlete.

In an example according to this aspect of the present invention, the sample obtained obtained from the athlete is a serum sample, a plasma sample or a urine sample.

In another example, the athlete is a human athlete or a non-human athlete selected from a horse, a camel or a dog.

In a further aspect of the present invention there is provided an article of manufacture for screening a test sample for the presence of a ligand, which ligand is capable of activating a steroid hormone receptor and eliciting a genomic response in a cell, the article of manufacture comprising a test kit as described herein together with instructions for how to detect the presence of a ligand in the sample.

In yet a further aspect of the present invention there is provided an article of manufacture for determining doping in an athlete, the article of manufacture comprising a test kit as described herein together with instructions for detecting the presence of a ligand in a sample derived from the athlete, wherein the presence of the ligand in the sample is indicative of doping in the athlete.

The various test kits and assays described herein each provide (i) a steroid hormone receptor inclusive of a ligand binding domain for binding a ligand that *may* be present in a sample to be tested and (ii) a nucleic acid response element comprising a protein binding domain which is bound by an activated steroid hormone receptor (or ligand-receptor complex; HR-L). The term "activated steroid hormone receptor" refers to a receptor-ligand complex, and may include various permutations of the HR-L structure (e.g. monomer, dimer, trimer *etc*). Importantly, the hormone response element contains binding motifs specific for the receptor-ligand complex. Accordingly, by combining the test kits and assays of the present invention with a sample of interest, detection of a ligand, which possesses the potential to bind to a steroid hormone receptor and elicit a steroid hormone genomic response, is possible.

The terms "receptor binding domain", "activated receptor binding domain", "hormone receptor binding domain", "activated hormone receptor binding domain", "receptor-ligand binding domain" and "hormone receptor-ligand binding domain" are used interchangeably to refer to the protein binding domain of the hormone response element that is bound by an activated hormone receptor or ligand-receptor complex, as defined herein.

In other examples, the inventions described herein find utility in the detection of performance enhancing pro/drugs (e.g. anabolic steroids) used in human as well as non-human athletes including race horses and dogs. In other examples, the inventions described herein have utility in screening foods and health food supplements for additives that may bind to a steroid hormone receptor and elicit a genomic response in a cell or do so following metabolic processing (i.e. in the case of so-called 'prodrugs').

The present invention further contemplates detection of one or more physiologically inactivate ligands from a test sample, which ligands are ultimately capable of activating steroid hormone receptors when converted to a physiologically active form. As such, the test kits, assays and methods as described herein further comprise steroid metabolism machinery that is capable of processing the ligand in such a way that it will activate its corresponding steroid hormone receptor. In this way, detection of physiologically inactive ligands (e.g. prohormones) from samples such as nutritional supplements is possible.

As such, the test kits, assays and methods described herein may further comprise steroid metabolism machinery sufficient to convert a ligand from a physiologically inactive form to a physiologically active form, or from a physiologically active form to a *more* physiologically active form or from a physiologically active form to a *less* physiologically active form, or from a physiologically active form to a physiologically inactive form. Only when the ligand is in a physiologically active form does it possess the ability to activate a steroid hormone receptor and elicit a genomic response. Accordingly, inclusion of steroid metabolism machinery in the test kits, assays and methods according to the present invention helps facilitate detection of physiologically inactive ligands from a test sample of interest, (e.g.) which ligands exist as pro-drugs (e.g. pro-hormones) and might otherwise evade detection using established methodologies. Furthermore, inclusion of steroid metabolism machinery in the test kits, assays and methods according to the present invention helps determine biological activity/potency of ligands necessary to show effect.

The data presented in Figure 17 further illustrate this point. Androstenedione (i.e. a androgen prohormone) was preincubated with S9 liver fraction before the reaction mix was extracted for steroids (i.e. to remove any potential non-specific ligands). The various test and control samples were then analysed for androgenic activity. The results demonstrate a statistically significant reduction in fluorescence of the reporter construct for Androstenedione+S9 liver treatment compared to the no-treatment control (i.e. Androstenedione-S9 liver), where the androgenic activity levels for the no treatment control approximated the vehicle control (i.e. methanol + T7; methanol + AR/HSP90).

The test kits, assays and methods described herein may further comprise a detection means for detecting binding between the receptor-ligand complex and the response element contained within the nucleic acid, as defined.

The test kits and assays according to the present invention are cell-free. This is particularly important since the molecular complexity of the assay systems are significantly reduced. For example, the absence of (i) a cell membrane structure which has the potential to create a thermodynamic sink for steroid hormone molecules and (ii) endogenous steroid hormone metabolism observed with cell based systems, provides for an assay system with enhanced sensitivity. Further, and advantageously, according to the test kits, assays and methods described herein, the relative amounts of essential structural elements (e.g. steroid hormone receptor and nucleic acid response element inclusive of one or more activated receptor binding domains) may be precisely controlled to provide enhanced assay functionality and increased sensitivity.

According to the methods described herein, the test result may be compared to a reference threshold in order to determine the absolute level of signal generated by a ligand present in a test sample. Indeed, Applicants observed non-specific binding and/or activation of the response element by non-ligand bound receptor. Accordingly, where it is desirable to perform a semi-quantitative analysis for any given test sample, the assays and methods described herein may be performed in the absence of test sample to first establish a reference threshold (e.g. in presence of ethanol acting as a negative control). Assay results obtained from a test sample may be then be compared to the reference threshold, to determine the absolute activity attributable to the ligand(s) present in a sample using a simple subtraction methodology.

The present invention further contemplates the use of the assays and test kits described herein to determine the potency of a test compound relative to a reference compound. According to the present invention, the term 'relative potency' is defined as the multiplier of biological activity of a test compound relative to a reference compound, as determined by normalizing the biological activity of the test compound to the reference compound.

The biological activity of the test and reference compounds may be determined using EC₅₀ or the concentration of compound that gives half the maximal response from a dose response curve for that particular compound. The dose response curve is generated by serially diluting the compound and measuring its steroid hormone receptor binding/activation profile. A plot of the measured activity (e.g. as measured by fluorescence) vs concentration of the compound (i.e. serial dilution of the compound generates a concentration range that is best presented on a log scale) is then made.

A person skilled in the art will recognize that a measure of relative potency of a test compound is *relative* to the reference compound used. In other words, the relative potency of a test compound is likely to differ depending on the reference compound to which its biological activity is normalized.

Where the relative potency is >1, the test compound invokes a higher measured biological activity in the assays compared to the reference compound. Where the relative potency is <1, the test compound invokes a lower measured biological activity in the assays compared to the reference compound. Where the relative potency =1, the test compound and the reference compound invoke equal biological activity in the assays.

Relative potency can also be used to determine the activation factor of a test compound in question. As used herein, activation factor relates the relative potency of a test compound determined in a yeast cell or using a yeast cell-free extract (i.e. which contains no metabolic machinery) to the relative potency of the same test compound determined in a mammalian cell or using a mammalian cell-free extract (i.e. which includes metabolic machinery) as a measure of relative activation between the two states of the test compound. An activation factor >1 means that the test compound has undergone metabolic conversion to a more physiologically active state in the presence of metabolic machinery in the assay.

Yet another advantage conferred by the test kits and assays according to the present invention is the relative ease of performance. In other words, performance of the test kits, assays and methods described herein does not require complex cell culture techniques, experienced laboratory technicians or convoluted laboratory testing equipment and analysis. This is particularly advantageous, because the test kits, assays and methods according to the present invention may be practiced by untrained personnel in the field following relatively simple testing procedures. Further, performance of the test kits, assays and methods may provide real time information (e.g.) when testing for performance enhancing substances in a sample taken from an athlete immediately prior to, or following, competition.

In other aspects of the present invention there is provided a test kit for screening a sample for the presence of a ligand, which ligand is capable of forming a complex with a steroid hormone receptor and eliciting a genomic response when in a cell, the test kit comprising:
(i) an androgen receptor that is capable of forming a ligand-receptor complex with a ligand from the test sample; or
(ii) an estrogen receptor that is capable of forming a ligand-receptor complex with a ligand from the test sample, wherein the estrogen receptor is estrogen receptor alpha or estrogen receptor beta; or
(iii) a progesterone receptor that is capable of forming a ligand-receptor complex with a ligand from the test sample, wherein the progesterone receptor is progesterone receptor A or progesterone receptor B; or
(iv) a mineralocorticoid receptor that is capable of forming a ligand-receptor complex with a ligand from the test sample; or
(v) a glucocorticoid receptor that is capable of forming a ligand-receptor complex with a ligand from the test sample; and
(vi) a nucleic acid molecule comprising:
   (a) a polymerase promoter sequence;
   (b) a response element that is capable of being bound by the receptor-ligand complex; and
   (c) a reporter construct
   where the response element (b) is located between the promoter sequence (a) and the reporter construct (c), and (a), (b) and (c) are operably linked; and

(iii) a single polypeptide polymerase,
wherein, the presence of a ligand in the sample is detected by measuring a reduction or inhibition in transcription of the reporter construct caused by binding of the ligand-receptor complex to the response element when the sample is combined with the test kit.

In other aspects of the present invention there is provided a test kit for screening a sample for the presence of a ligand, which ligand is capable of forming a complex with a steroid hormone receptor and eliciting a genomic response when in a cell, the test kit comprising:
(i) an androgen receptor that is capable of forming a ligand-receptor complex with a ligand from the test sample; or
(ii) an estrogen receptor that is capable of forming a ligand-receptor complex with a ligand from the test sample, wherein the estrogen receptor is estrogen receptor alpha or estrogen receptor beta; or
(iii) a progesterone receptor that is capable of forming a ligand-receptor complex with a ligand from the test sample, wherein the progesterone receptor is progesterone receptor A or progesterone receptor B; or
(iv) a mineralocorticoid receptor that is capable of forming a ligand-receptor complex with a ligand from the test sample; or
(v) a glucocorticoid receptor that is capable of forming a ligand-receptor complex with a ligand from the test sample; and
(vi) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(vii) a nucleic acid molecule comprising:
   (a) a polymerase promoter sequence;
   (b) a response element that is capable of being bound by the receptor-ligand complex; and
   (c) a reporter construct where the response element (b) is located between the promoter sequence (a) and the reporter construct (c), and (a), (b) and (c) are operably linked; and
(iii) a single polypeptide polymerase,
wherein, the presence of a ligand in the sample is detected by measuring a reduction or inhibition in transcription of the reporter construct caused by binding of the ligand-receptor complex to the response element when the sample is combined with the test kit.

In yet further aspects of the present invention there is provided a test kit for screening a test sample for the presence of a ligand capable of eliciting a steroid hormone genomic response, the test kit comprising:
(i) an androgen receptor that is capable of forming a ligand-receptor complex with a ligand from the test sample; or
(ii) an estrogen receptor that is capable of forming a ligand-receptor complex with a ligand from the test sample, wherein the estrogen receptor is estrogen receptor alpha or estrogen receptor beta; or
(iii) a progesterone receptor that is capable of forming a ligand-receptor complex with a ligand from the test sample, wherein the progesterone receptor is progesterone receptor A or progesterone receptor B; or
(iv) a mineralocorticoid receptor that is capable of forming a ligand-receptor complex with a ligand from the test sample; or
(v) a glucocorticoid receptor that is capable of forming a ligand-receptor complex with a ligand from the test sample; and
(vi) a nucleic acid molecule comprising:
   (a) a polymerase promoter sequence;
   (b) a response element that is capable of being bound by the receptor-ligand complex as defined in any of (i) to (v) above; and
   (c) a reporter construct,
   where the response element (b) is located between the promoter sequence (a) and the reporter construct (c), and (a), (b) and (c) are operably linked; and
(vii) a single polypeptide polymerase; and
(viii) nucleoside triphosphates;
wherein, the presence of a ligand in the sample is detected by measuring a reduction or inhibition in transcription of the reporter construct caused by binding of the ligand-receptor complex to the response element when the sample is combined with the test kit.

In yet further aspects of the present invention there is provided a test kit for screening a test sample for the presence of a ligand capable of eliciting a steroid hormone genomic response, the test kit comprising:
(i) an androgen receptor that is capable of forming a ligand-receptor complex with a ligand from the test sample; or
(ii) an estrogen receptor that is capable of forming a ligand-receptor complex with a ligand from the test sample, wherein the estrogen receptor is estrogen receptor alpha or estrogen receptor beta; or
(iii) a progesterone receptor that is capable of forming a ligand-receptor complex with a ligand from the test sample, wherein the progesterone receptor is progesterone receptor A or progesterone receptor B; or
(iv) a mineralocorticoid receptor that is capable of forming a ligand-receptor complex with a ligand from the test sample; or
(v) a glucocorticoid receptor that is capable of forming a ligand-receptor complex with a ligand from the test sample; and
(vi) a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52; and
(vii) a nucleic acid molecule comprising:
   (a) a polymerase promoter sequence;
   (b) a response element that is capable of being bound by the receptor-ligand complex as defined in any of (i) to (v) above; and
   (c) a reporter construct,
   where the response element (b) is located between the promoter sequence (a) and the reporter construct (c), and (a), (b) and (c) are operably linked; and
(viii) a single polypeptide polymerase; and
(ix) nucleoside triphosphates;
wherein, the presence of a ligand in the sample is detected by measuring a reduction or inhibition in transcription of the reporter construct caused by binding of the ligand-receptor complex to the response element when the sample is combined with the test kit.

In certain examples according to the assays, methods and test kits of the present invention, the nucleic acid molecules include one or more copies of various components of the nucleic acid, including the response element or reporter construct. For example, the reporter constructs or the nucleic acid molecules may include a single copy or multiple copies of the nucleic acid response elements including, but not limited to, duplicate copies, triplicate copies, quadruple copies etc.

In another example of the present invention, the steroid hormone receptor is purified from a cell, or is derived from a cell-based hormone receptor through recombinant cloning, expression and purification. In a further example, the steroid hormone receptor is synthetic, and its sequence modeled on, or evolved from, endogenous steroid hormone receptor sequences known in the art.

A person skilled in the art would also recognize that any steroid hormone receptor may be employed in the test kits, assays and methods of the present invention, provided that it retains the ability to bind to, and be activated by, a ligand of interest for detection. This includes, steroid hormone receptors, based on endogenous cellular forms, as well as recombinant or synthetic forms.

As such, the test kits, assays and methods according to the present invention may be configured to screen/detect any ligand that elicits a steroid hormone genomic response. However, a person skilled in the art will recognise that, according to the various assays concepts described herein, detection of different hormone classes (i.e. ligands) requires the format of the test kits, assays and methods to be properly configured and optimized. For example, detection of a ligand that binds to and activates an androgen receptor, such as testosterone as well as other testosterone-like hormones, requires test kits/assays comprising androgen receptor together with an androgen response element capable of binding to an activated androgen-receptor complex etc.

Designer steroids and non-steroidal anabolic drugs pose a significant and growing challenge for anti-doping laboratories. First identified in the early 2000s with the detection of tetrahydrogestrinone and madol, the threat posed by designer anabolic drugs has rapidly increased to include numerous potential agents. These synthetically-derived anabolic drugs are designed to evade detection or legal controls with respect to both manufacture and supply, and many are widely available on the internet where they are sold as so-called "supplements".

Mass spectrometry remains the primary technology for the identification of known illicit steroid hormones and non-steroid anabolic drugs in biological samples and/or supplements. Despite its sensitivity and specificity, mass spectrometry remains limited by requiring prior knowledge of the steroid and non-steroid anabolic drug's chemical structures for detection. Moreover, mass spectrometry fails to provide information about the biological activity of the anabolic drugs detected, and is unable to differentiate between bioactive and inactive molecules. This is information that is required for legal prosecution of athletes, coaches, trainers, managers and manufacturers.

In recent years, yeast and mammalian cell-based *in vitro* androgen bioassays have been used to detect the presence of novel synthetic androgens, the androgenic potential of progestins as well as androgens, pro-androgens, designer androgens and designer non-steroid anabolic drugs in supplements. However, these assays suffer limitations associated with molecular complexity, as described elsewhere herein, and require technical skills that are both molecular and cellular in nature, are time consuming, labour intensive and expensive. As such, it is not feasible to consider the assays in their present form for inclusion in routine screening. In other words, yeast and mammalian cell-based assays suffer significant limitations because they are not high throughput or cost effective.

Advantageously, the present invention provides activity based test kits, assays and methods that work fundamentally on the principle of steroid hormone receptor activation. By detecting steroid hormone receptor activation by a ligand present within a sample to be tested, the present invention provides cell-free test kits, assays and methods that do not rely on structural knowledge of the ligand(s) being interrogated, can readily distinguish between the presence of biologically active and inactive ligands, and provide cost-effective, reliable and reproducible systems that do not require complex laboratory equipment or particular expertise to perform.

Accordingly, in an example according to the test kits, assays and methods described herein, the ligand is a performance enhancing designer drug and/or steroid.

In another example according to the test kits, assays and methods described herein, the ligand is of an unknown chemical structure.

In a further example according to the test kits, assays and methods described herein, the ligand is of a previously unknown chemical structure.

The present invention further contemplates use of the test kits, assays and methods as described herein for detecting antagonists of a target ligand by screening a sample of interest for a compound that will prevent binding of the ligand to its steroid hormone receptor such that it no longer activates the receptor and elicits a genomic response.

This is particularly useful when there is a need to screen for antagonists that block steroid hormone receptor activation (e.g.) as potential therapeutics for the treatment of endocrine and non-endocrine cancers. For example, the activity based test kits, methods and assays comprising one or more estrogen receptors according to the present invention can be used to screen compound libraries for the presence of antagonists or to monitor the loss of estrogen receptor activation in breast cancer tissue or blood in patients on cancer therapy.

In yet a further example according to all aspects of the test kits, assays and methods described herein, the biological sample is derived from an animal selected from the group consisting of equine, canine, camelid, bovine, porcine, ovine, caprine, avian, simian, murine, leporine, cervine, piscine, salmonid, primate, and human.

In yet a further example according to all aspects of the test kits, assays and methods described herein, the test sample is derived from biological material selected from the group consisting of urine, saliva, stool, hair, tissues including, but not limited to, blood (plasma and serum), muscle, tumors, semen, etc.

In yet a further example according to all aspects of the test kits, assays and methods described herein, the test sample is derived from a food selected from the group consisting of vegetable, meat, beverage including but not limited to sports drink and milk, supplements including, but not limited to, food supplements and sports supplements, nutritional supplements, herbal extracts, etc.

In yet a further example according to all aspects of the test kits, assays and methods described herein, the test sample is derived from a medication selected from the group consisting of drug, tonic, syrup, pill, lozenge, cream, spray and gel.

In yet a further example according to all aspects of the test kits, assays and methods described herein, the sample is derived from the environment selected from the group consisting of liquid, water, soil, textile including, but not limited to, plastics and mineral.

The information presented in Example 6 read in conjunction with Figures 14-16, demonstrates that the test kits and assay methods were used to detect testosterone (as an exemplary steroid hormone ligand) from biological matricies such as serum derived from calves and horses (Fig. 14), as well urine obtained from colts (Figs. 15 and 16). These data reinforce the utility of the test kits and assay methods according to the present invention in the field, for example, in determining the doping status of human and equine athletes trackside, or for performing analysis on food supplements as part of export/import quality control.

Accordingly, in another example, the sample is a biological sample. In a related example, the biological sample is a body fluid sample, including but not limited to, blood, plasma, serum, saliva, interstitial fluid, semen and urine.

In another example, the sample derived from a plant, including but not limited to, leaf, flower, stem, bark, root, bud, pod, pollen and seed.

In another example, the sample is derived from an animal including, but not limited to, an equine animal, a canine animal, a dromedary animal, a bovine animal, a porcine animal, an ovine animal, a caprine animal, an avian animal, a simian animal, a murine animal, a leporine animal, a cervine animal, a piscine animal, a salmonid animal, a primate animal, and a human animal.

In another example, the test sample is a non-biological sample. In a related example, the non-biological sample includes, but is not limited to, a liquid sample including water, a soil sample, a textile sample including but not limited to plastics, a mineral sample, a food sample and a medication.

Examples of a food sample includes, but is not limited to, vegetables, meats, beverages, supplements and herbal extracts.

Examples of a medication includes, but is not limited to, drugs, tonics, syrups, pills, lozenges, creams, sprays and gels.

The invention is further described with reference to the following examples. It will be appreciated that the invention as claimed is not intended to be limited in any way by these examples.

### EXAMPLES

The information and data which follows demonstrates various prototype assays with respect to the detection of ligands that bind to and activate androgen receptors including (e.g.) Testosterone and Dihydrotestosterone, or ligands that bind to and activate estrogen receptors including (e.g.) Estradiol. These Examples are used to illustrate the activity assay platform described and claimed herein, where the assay concepts and principles exemplified by the detection of ligands that bind to androgen receptors or ligands that bind to estrogen receptors (i.e. ER-α, and ER-β) would apply equally to the detection of other receptor ligands of interest including, without limitation, ligands that bind to progesterone receptor including but not limited to progesterone, ligands that bind to mineralocorticoid receptor including but not limited to aldosterone, and ligands that bind to the glucocorticoid receptor including but not limited to cortisol.

### EXAMPLE 1

### ANDROGEN ASSAY PROTOTYPE 1: ASSAY ARCHITECTURE & RESULTS

### 1.1 In Vitro Transcription Platform

Applicants initially developed an in vitro transcription platform include a DNA construct that encodes a T7 RNA consensus promoter sequence upstream of a tandem array of 3X androgen response elements (ARE) upstream of an RNA aptamer sequence for Mango II, combined with recombinant androgen receptor (AR), recombinant heat shock protein 90 (HSP90), T7 RNA polymerase, nucleoside triphosphates and a transcription buffer. The T7 promoter will drive a high level of RNA aptamer expression that will be detected by binding to fluorophore, Thiazole Orange 1 - biotin (TO1). Inhibition of T7-driven aptamer expression will occur when ARE is bound by ligand-activated AR.

### Androgen response element (ARE)

- The androgen response element tested in these experiments was a tandem array of 3xARE

### Androgen receptor (AR)

- Commercial recombinant AR has been tested from Sigma-Aldrich

### T7 RNA polymerase

- The MegaScript kit from ThermoFisher was used in these studies, as a source of T7 RNA polymerase, and necessary buffers and nucleotide triphosphates.

### 1.2 Description of ARE-mediated transcription/translation

Natural androgen signaling starts with the diffusion of an androgenic molecule into the cell where it binds to the AR being held in an inactive state in the cytoplasm, bound to heat shock protein 90 (HSP90). Upon binding the androgenic molecule (or ligand), AR undergoes a conformational change that releases HSP90, and exposes nuclear localization and dimerization sites. The ligand-AR complex is targeted for translocation to the nucleus where AR binds to ARE sites in the DNA and RNA polymerase II holoenzyme assembles and initiates transcription of AR-regulated genes. Transcription of a gene by RNA polymerase II produces an mRNA transcript that, in turn, acts as the template for the ribosome machinery to make a protein.

Exploiting this natural biology, the experiments described now show testosterone (a natural androgen)- activated AR decreases T7-mediated transcription, as ARE has been placed *downstream* of the T7 promoter (and transcription initiation site) so when testosterone-liganded AR binds to the ARE, there is a reduction/inhibition in T7-mediated transcription.

Figure 1 illustrates a schematic of ARE-mediated blocking of T7 RNA polymerase-mediated transcription.

In the presence of ligand, such as the natural androgen, testosterone, AR binds to ARE and inhibits transcription by T7 polymerase. In this state, less RNA Mango II aptamer is formed.

In the absence of ligand, AR is not activated to bind to ARE, and therefore the DNA is free from an obstructive protein. T7 proceeds along the DNA construct to generate RNA Mango II aptamer, which is subsequently detected by TO1-B binding and fluorescence.

In reference to the result presented in Figures 2 and 3, testosterone-activation of AR blocking of transcription was investigated. No AR shows full T7-mediated transcription and RNA Mango II aptamer expression. This is reduced in the presence of AR, and further reduced when AR is activated by 11.5 ng/ml testosterone.

In reference to Figure 4, it was next investigated if HSP90 could inhibit the baseline activation of AR. AR is activated by conformational change through activation of region activation factor-1 (AF-1) via testosterone, or via auto-activation of region activator factor-2 (AF-2). For AR, AF-2 is very active and can represent up to 50% of AR activity. To suppress AF-2 auto-activation, HSP90 was added to keep AR in an inactivated state that would not bind to ARE in the absence of ligand. In the presence of ligand, however, HSP90 should competitively dissociate from the AR, and the liganded-AR should bind to the ARE.

### EXAMPLE 2:

### ANDROGEN ASSAY PROTOTYPE 2: ASSAY ARCHITECTURE & RESULTS

The concept of the Androgen Assay Prototype 2 is that a single protein RNA polymerase, such as T7 RNA polymerase, binds to its promoter sequence on a DNA template. The DNA template encodes the RNA aptamer Mango II sequence downstream of the promoter. A hormone response element (HRE) is located between the T7 promoter and the Mango II sequence. When a steroid hormone receptor (SHR) is added to the T7 *in vitro* transcription (IVT) reaction mix and activated by a receptor-specific ligand, the SHR binds to the HRE on the DNA template and in this bound position physically inhibits T7 RNA polymerase from transcribing the DNA into the RNA, and therefore no Mango II aptamer is formed. The formation of Mango II is detected by adding a specific fluorophore, such as TO1-biotin, to the reaction mix which binds to the Mango II aptamer. Upon binding to the Mango II aptamer, TO1 emits an excitation wavelength at 535 nm wavelength when excited by 510 nm wavelength. Fluorescence is measured using a standard fluorimeter.

### 2.1 DNA sequences used in development of Androgen Assay Prototype 2

The following experiments use AR or ERα as the example SHR and the ARE or ERE as the example HRE.

The key step that underpins T7-mediated *in vitro* transcription is for the recognition of its promoter sequence in the DNA template. To then measure that T7 transcription has occurred the DNA sequence can encode a reporter enzyme (protein) or a reporter RNA (e.g. aptamer). In the following examples, the DNA sequence encoded a reporter RNA (Mango II).

Commercial DNA fragment synthesis was used to generate a series of DNA templates that encoded the (1) T7 initiator sequence, (2) ARE, (3) Mango II RNA aptamer with F30 scaffold. These DNA fragments were cloned into a plasmid and amplified using transformed *E. coli.* Subsequent plasmid extraction, purification and linearization provided the final DNA template for Prototype Assay. Other examples where transcription factors have blocked T7 activity show that ~15 bp between the T7 initiator sequence and the transcription factor site is optimal for blocking T7 progress. Therefore, a 15 bp filler sequence was included in the DNA fragment.

**Table 2: Sequences Used in Prototype 2 for Detection of Androgenic Ligands**

| **Component** | **Sequence** |
|---|---|
| T7 initiator sequence | TAATACGACTCACTATAG (SEQ ID NO: 1) |
| 15bp filler | ACTCTGGAGGAA (SEQ ID NO: 74) |
| 3XtandemARE | AAGCTTAGAACAGTTTGTAACGAGCTCGTTACAAACTGTTCTAGC TCGTTACAAACTGTTCTAAGCT (SEQ ID NO: 75) |
| Primary ARE | TGGAGAACAGCCTGTTCTCCA (SEQ ID NO: 76) |
| MangoIIF30scaffold* | TTGCCATGTGTATGTGGGTACGAAGGAGAGGAGAGGAAGAGGAG AGTACCCACATACTCTGATGATCCTTCGGGATCATTCATGGCAATC TAGGA (SEQ ID NO: 77) |
| MangoII | TACGAAGGAGAGGAGAGGAAGAGGAGAGTA (SEQ ID NO: 78) |

| | |
|---|---|
| *single underline region, Mango II | |

### 2.2 Testosterone-activated AR is able to suppress T7-mediated expression of RNA aptamer, Mango II

The SHR-HRE-RNA aptamer reaction to detect a SHR ligand hinges on the blockade of T7 transcription by an SHR bound to a hormone response element. In the following examples, AR and ARE were used to represent SHR and HRE.

**Table 3: Reaction components required for the AR/HSP90-ARE reaction with T7-generated Mango II as readout**

| **Component** | **Concentration/Activity per reaction*** |
|---|---|
| T7 RNA polymerase | 0.5 µl |
| T7 ARE ManqoII DNA template | 100 ng |
| T7 reaction buffer** | 2.5 µl |
| AR | 50 ng |
| HSP90 | 100 ng |
| Steroid hormone | 250 µM |
| Mango II-specific fluorophore | 100 nM |

| | |
|---|---|
| *concentrations for the initial experiments were based on previous work (e.g. FRET assays for AR/HSP90 or other cell-free assays established in the inventor's laboratory) or manufacturer's protocols (DNA template concentration, enzyme, and fluorophore) **T7 reaction buffer (eg. NTP buffer from HiScribe kit [ThermoFisher Scientific] or 10X buffer supplied with T7 enzyme or home-made buffer) | |

The reactions were assembled and initiated with the addition of T7 RNA polymerase and incubated for 150 mins at 37°C. During this time, RNA Mango II aptamer was generated. The detection of Mango II was by the addition of fluorophore, thiazole orange (TO1) and measuring output with fluorimeter, excitation 510 nm and emission 535 nm. Figure 5 shows that testosterone-activated AR is able to reduce the amount of T7-generated RNA aptamer Mango II in a dose-dependent manner. Testosterone represents the most abundant endogenous circulating androgen in the body. Testosterone can be converted in peripheral tissues (e.g. gonads) to dihydrotestosterone (DHT). DHT was also found to activate AR-regulated reduction in T7-generation of Mango II.

### 2.3 Titration of the Steroid Hormone Receptor

As above, AR was used as the example steroid hormone receptor in the following experiments.

In the above experiment, and as shown in Table 3, the initial AR concentration was based on previous cell-free assays established in the inventors' laboratory. The reduction in T7-mediated Mango II generation is dependent on there being sufficient AR to block all the T7 enzyme molecules currently engaged with, and active on, the DNA templates. This means that both the ratios of AR to T7 and AR to DNA template are important. The level of AR, however, needs to be considered in the context of an optimal level of T7 and/or DNA template that supports sufficient Mango II generation required for detecting changes in fluorescence.

In the next series of experiments, the concentration of AR per reaction was altered to show the effects on Δ[Mango II].

**Table 4: Titration of AR**

| **Component** | **Concentration per reaction** | | | |
|---|---|---|---|---|
| T7 RNA polymerase | 0.5 µl | 0.5 µl | 0.5 µl | 0.5 µl |
| T7 ARE MangoII DNA template | 100 ng | 100 ng | 100 ng | 100 ng |
| T7 reaction buffer | 2.5 µl | 2.5 µl | 2.5 µl | 2.5 µl |
| **AR** | **14.2 ng** | **25 ng** | **50 ng** | **100 ng** |
| HSP90 | 100 ng | 100 ng | 100 ng | 100 ng |
| Steroid hormone | 250 µM | 250 µM | 250 µM | 250 µM |
| Mango II-specific fluorophore | 100 nM | 100 nM | 100 nM | 100 nM |

Using the 50ng AR reaction as the "standard" reaction, the number of molecules of AR is 2.737e¹¹ (454.55 fmol or 22.72 nM) to the number of DNA molecules at 3.798e¹⁰ (63.06 fmol, or 3.15 nM) resulting in an excess ratio of AR:DNA of 7.2:1. Doubling the AR concentration to 100ng, doubles the ratio to 14.4:1. Halving the AR concentration to 25 ng, halves the ratio to 3.6:1, and further again for the 14.2 ng AR to 1.8:1.

The data in Figure 6 shows that the AR:DNA ratio of 7.2:1 or greater produces the greater ΔMango II. The lower ratios still show ΔMango II but the effect size is reduced. Notably the jump in ratio from 7.2 to 14.4 showed no improvement in effect size on ΔMango II suggesting that at 7.2:1 the excess of AR to DNA is sufficient for blocking T7 activity, and having a further excess creates redundancy.

When considering SHR activation by steroid hormones, it is also necessary to examine the ratio of HSP90 to SHR, as an excess of HSP90 will block activation of the SHR, especially at low concentrations of ligand, while insufficient HSP90 will allow autoactivation of the SHR. Again, using AR as the representative SHR, in the next set of experiments, the ratio of HSP90 to AR was altered to scrutinize effects on T7-generation of Mango II by testosterone.

**Table 5: Titration of the HSP90:AR ratio**

| **Component** | **Concentration per reaction** | | | | | | |
|---|---|---|---|---|---|---|---|
| T7 RNA polymerase | 0.5 µl | 0.5 µl | 0.5 µl | 0.5 µl | 0.5 µl | 0.5 µl | 0.5 µl |
| T7 ARE MangoII DNA template | 100 ng | 100 ng | 100 ng | 100 ng | 100 ng | 100 ng | 100 ng |
| T7 reaction buffer | 2.5 µl | 2.5 µl | 2.5 µl | 2.5 µl | 2.5 µl | 2.5 µl | 2.5 µl |
| **AR** | **14.5 ng** | **25 ng** | **50 ng** | **100 ng** | **100 ng** | **50 ng** | **25 ng** |
| **HSP90** | **100 ng** | **100 ng** | **100 ng** | **200 ng** | **100 ng** | **50 ng** | **25 ng** |
| Steroid hormone | 250 µM | 250 µM | 250 µM | 250 µM | 250 µM | 250µM | 250 µM |
| Mango II-specific fluorophore | 100 nM | 100 nM | 100 nM | 100 nM | 100 nM | 100 nM | 100 nM |

Figure 7 shows that a standard reaction of 50ng AR and 100ng HSP90 with a ratio of HSP90:AR of 2.44:1 was optimal, however other ratios could achieve the same level of ΔMango II. For example, a 100 ng HSP90 corresponding to 6.69e¹¹ molecules (1.11 pmol or 55.5 nM) and 100 ng AR corresponding to 5.47e¹¹ molecules (909.09 fmol or 45.5 nM) or ratio of 1.22:1 also showed good suppression of T7-mediated generation of Mango II. Equally, AR (25 ng) and HSP90(100 ng) representing 1.37e¹¹ molecules (227.27 fmol or 11.4 nM) and 6.69e¹¹ molecules (1.11 pmol or 55.5 nM) respectively, or ratio of 4.88:1 showed a ΔMangoII that was not different to a ratio of 2.44:1. However, a 9.76:1 ratio showed reduced ΔMangoII, as did 1.22:1 ratios where AR concentration was < 100ng.

Importantly, the data from Figures 6 and 7 highlights a unique ability to stoichiometrically define a biological reaction.

AR is most effective at binding to an ARE and blocking T7 progress when the AR:DNA ratio is ≥ 7.2:1. AR is most effective at being activated by ligand and binding to an ARE when the HSP90:AR ratio is between 1.22:1 and 4.88:1.

### 2.4 Manipulating the HRE

The data so far has used a 3X ARE sequence commonly used in cell-based AR bioassays (see Table 2). However, the primary ARE that has been identified is of the sequence AGAACAgccTGTTCT. Considering the functioning of this assay whereby T7 enzyme is physically blocked by AR, it may not be necessary for the presence of 3X ARE sequences. In the following test, a single, but primary in sequence, ARE was cloned into the T7 DNA template. Figure 8 clearly shows that the single ARE was as effective as the 3X ARE sequence with activated AR blocking T7 RNA polymerase.

### 2.5 Titrating the DNA template

The DNA template is a critical component of the assay and needs to be at a concentration that supports T7 transcription while also not being in excess so that the number of AR molecules present can sterically hinder transcription of the reporter construct by T7 enzyme.

In the next series of experiments, the DNA template was titrated holding AR and T7 constant. This provided insight into the number of DNA molecules required to support transcription and to maintain AR blockade. Figure 9 data shows that when the concentration of AR was 50 ng (2.737e¹¹ molecules, 454.55 fmol or 22.72 nM) and the DNA concentration was 100 ng (3.798e¹⁰, 63.06 fmol or 3.15 nM) there was good detection of ΔMango II, representing an AR:DNA ratio of 7.2:1. Increasing the amount of excess AR to DNA by lowering DNA concentration to test ratios of 14.4:1, 17.28:1 and 28.8:1 had no effect on improving ΔMango II. Thus, an excess ratio of 7.2:1 achieves maximal effect. Note, that reducing DNA template to 6.46e⁹ or 3.398e⁹ did affect ΔMango II, because there was a dramatic decrease in T7-mediated fluorescence output (data not shown). Thus, 9.495e⁹ molecules of double-stranded DNA is the minimum level required for a successful reaction, while an excess of AR:DNA must be maintained above 7.2:1. Going beyond 7.2 with addition of more AR creates redundancy, while decreasing DNA to increase the ratio risks T7-mediated transcription *per se.*

Data from Figure 9 has continued to define a biologically effective, but synthetically achieved SHR reaction. Through the stoichiometric analysis of individual components, the experiments have confirmed that AR is most effective at binding to an ARE and blocking T7 progress when the AR:DNA ratio is ≥ 7.2:1. However, when making up this ratio it is necessary to lock the DNA template concentration at a minimum of 9.495e⁹ molecules of double-stranded DNA otherwise the base level of transcription is compromised.

### 2.5 Titrating T7 polymerase

The final component of the Androgen Assay Prototype 2 that needs to be considered to allow a fully defined stoichiometric reaction that can mimic steroid hormone receptor biology is the T7 enzyme itself. T7 RNA polymerase is used in this reaction to generate the reporter, which in these examples is the RNA aptamer, Mango II.

The amount of T7 enzyme is critical to maintain the fluorescent readout within a spectrum that will allow an optimal dynamic range. T7 is an enzyme so it is not just concentration that is an important factor, but also activity. The next series of experiments showed the effect of altering T7 activity on ΔMango II.

In Figure 10, data shows T7-generated MangoII:TO1B fluorescence levels as the T7 units are titrated from 50U to 10U. It is noted that in the enzyme range of 40-10U there is no clear difference in fluorescence measured with all measurements close to 200000 indicating a clear detection baseline, at which point sensitivity is not high enough to measure change in output. This suggests that in order to measure ΔMangoII, the fluorescence measurement must be greater than 200000.

To further demonstrate the effect of minimal fluorescence level, T7 (50U) or T7 (100U) was used to generate Mango II RNA aptamer, in a reaction blocked with testosterone-induced AR, or as control ethanol. The data in Figure 11 shows that if 50U is used the fluorescence is ~ 250000-300000, testosterone-activation shows ~9% in ΔMango II however if the fluorescence is in the range >600000 the same reaction shows a ~16% ΔMango II. Thus, the data highlights that T7 activity must be in the range that produces MangoII-TO1B fluorescence > 300000. The exact activity or units will be dependent on batch-to-batch and/or supplier-to-supplier differences in the specific activity of recombinant T7 RNA polymerase.

### EXAMPLE 3:

### ESTROGEN ASSAY PROTOTYPE 3: ASSAY ARCHITECTURE & RESULTS

In the above examples, AR/ARE was used as the example SHR/HRE. The following series of experiments used the defined reaction stoichiometry established for AR/ARE to show the applicability of the test to other SHRs, in this case ERα. The results presented below demonstrate that estradiol-activated ERα is able to suppress T7-mediated expression of RNA aptamer, Mango II.

**Table 6: Sequences Used in Prototype 3 for Detection of Estrogenic Ligands**

| **Component** | **Sequence** |
|---|---|
| T7 initiator sequence | TAATACGACTCACTATAG (SEQ ID NO: 1) |
| 15bp filler | ACTCTGGAGGAA (SEQ ID NO: 74) |
| Primary ERE | CAGGTCAGCATGACCTG (SEQ ID NO: 79) |
| MangoIIF30scaffold* | TTGCCATGTGTATGTGGGTACGAAGGAGAGGAGAGGAAGAGGAG AGTACCCACATACTCTGATGATCCTTCGGGATCATTCATGGCAATC TAGGA (SEQ ID NO: 77) |
| Mango II | TACGAAGGAGAGGAGAGGAAGAGGAGAGTA (SEQ ID NO: 78) |

| | |
|---|---|
| *single underline region, Mango II | |

The standard AR/ARE conditions that proved to be a successful detection test for testosterone were used, however AR was replaced with ERα and a DNA template encoding a single ERE replaced the ARE DNA template (Table 6). Figure 12 shows that replacing AR with ERα (50 ng) in combination with HSP90 (100 ng) and activating with 5 µM estradiol (E2) led to a reduced MangoII:TO1 output. The ΔMango II was ~60%. When considering ratios, ERα at 68kDa is smaller than AR (110kDa) and therefore the number of molecules added for weight was 4.428e11 (735.20 fmol or 36.8 nM). The ratio of HSP90:ERα therefore was 6.69e¹¹:4.428e¹¹ or 1.51:1 and ERα:DNA 4.428e¹¹:3.798e¹⁰ or 11.66:1. Both of these were in the range found to be successful ratios for detecting androgens with the AR version of the SHR/HRE test.

**Table 7: ERα/ERE reaction**

| **Component** | **Concentration per reaction** |
|---|---|
| T7 RNA polymerase | 0.5 µl or 100U |
| T7 ERE MangoII DNA template | 100 ng |
| T7 reaction buffer | 2.5 µl |
| ERα | 50 ng |
| HSP90 | 100 ng |
| Mango II-specific fluorophore | 100 nM |

The importance of the ERα:ERE reactions for detection of an estrogen is two-fold. Firstly, it shows the simplicity in switching out the essential test components from AR/ARE DNA template to an ER/ERE DNA template. Secondly, it shows the defined stoichiometric reaction established for AR/ARE that mimics androgen biology by ligand binding to an androgen receptor, becoming displaced from HSP90, and binding to an ARE is translatable to a second steroid hormone receptor/steroid response element combination.

The data shows that the test is able to recapitulate steroid hormone biology in a cell-free manner and in such a way that every component can be defined- a truly *in situ* test. This is unlike the situation *in vitro* when using cell-based bioassays or cell-free bioassays based on nuclear extracts providing the holoenzyme RNA polymerase II. In the case of cell-based bioassays, the levels of SHR, HSP90, DNA template, and RNA polymerase II cannot be defined at all because they are influenced by the expression pattern of the cell. Cell-free bioassays based on nuclear extracts can, in part, define the stoichiometry of a reaction by describing SHR, HSP90 and HRE levels, however are unable to define the RNA polymerase level. RNA polymerase II is a holoenzyme, made up of several subunits or proteins, and therefore can only be supplied in the form of a nuclear extract. The nuclear extract *is undefined* in what other proteins are present. In this single polypeptide RNA polymerase form of the assay, the stoichiometry of the reaction can be fully defined and the data shows that such reactions can by synthetically manipulated to mimic the natural biology of steroid hormone receptors.

### EXAMPLE 4

### LIGAND-SHR/HRE STOICHIOMETRIC REACTIONS FOR PROTOTYPE ASSAYS

The data presented in Examples 2 and 3 has revealed defined stoichiometry that mimics steroid hormone receptor biology with ligand binding to a specific receptor thereby the receptor displaces from HSP90 and binds to a steroid response element - the classical steroid hormone genomic response. In nature, the SHR will activate or repress the expression of the target gene. In the Prototype Assays described herein, binding of the liganded-SHR represses expression of the reporter molecule.

**Table 8: Ratio of molecules providing optimized output for Prototype Assays**

| **Components** | **Ratio** | |
|---|---|---|
| | **Androgen Assay Prototype 2** | **Figure (data)** |
| **SHR:DNA template** | 6:1-14.4:1 (>8 superfluous) | Fig. 6,9 & 12 |
| **SHR:HSP90** | 1.22:1-4.88:1 | Fig. 7 & 12 |
| **T7 activity (TO1B fluorescence readout)** | >600000 optimal (50-100U T7), >200000 threshold (25U T7) | Fig. 10, 11 |
| **HRE (copy number)** | 1X | Fig. 8 |

**Table 9: Template showing example stoichiometry of an SHR/HSP90/HRE reaction**

| **Components** | **Amount Androgen Assay Prototype 2** | **Concentration (nM)** |
|---|---|---|
| **SHR (# molecules)** | 2.737e¹¹-5.474e¹¹ | 22.7-45.5 |
| **HSP90 (#molecules)** | 6.69e¹¹ | 55.5 |
| **DNA template** | 9.459e⁹-3.798e¹⁰ | 0.79-3.15 |
| **T7 RNA polymerase*** | X units or µl | N/A |

| | | |
|---|---|---|
| *X defined by the volume/units (supplier dependent) of T7 RNA polymerase required to generate Mango II:TO1B fluorescence of >600000 units. Absolute baseline threshold of activity > 200000 fluorescence. | | |

### EXAMPLE 5

### ANDROGEN ASSAY PROTOTYPE 2 DETECTS ANDROGENIC MOLECULES

Androgenic molecules are primarily steroid hormones. Testosterone and dihydrotestosterone are the most abundant endogenous androgens. Based on their structures, synthetic androgenic anabolic steroids (AAS) have been designed and marketed. AAS are the most commonly abused performance enhancing drug in athletes, human and animal alike. Another class of androgenic molecules that have been synthetically derived are the selective androgen receptor modulators or SARMs. Like AAS, SARMs are abused by athletes. Both AAS and SARMs differ in their structures, with a great variety of different side groups and backbones. This next series of experiments tested whether the AR/HSP90-ARE Prototype Assay was able to detect different AAS and SARMs.

**Table 10: AAS and SARMs tested with Androgen Assay Prototype 2**

| **Androgenic compound** | **Nature of compound** |
|---|---|
| Testosterone | Endogenous |
| Dihydrotestosterone | Endogenous |
| 17α-trenbolone | AAS |
| 17β-trenbolone | AAS |
| TRENA | AAS-designer steroid-internet sourced |
| Altrenogest | Progestin, newly described androgen |
| Trendione | AAS |
| Nandrolone | AAS |
| Boldenone | AAS |
| 93746 | SARM |
| BMS | SARM |
| LDG | SARM |
| ACP105 | SARM |
| YK-11 | SARM |
| Andarine | SARM |
| Ligandrol | SARM |
| Ostarine | SARM |

Figure 13 shows that the assay was able to detect a variety of AAS and SARMs.

### EXAMPLE 6

### ANDROGEN ASSAY PROTOTYPE 2 DETECTS ANDROGENIC MOLECULES IN A BIOLOGICAL MATRIX

Androgen Assay Prototype 2 was next tested for its ability to detect testosterone when present in a biological matrix, such as serum or plasma. First, it was necessary to demonstrate that T7 RNA polymerase continued to operate in the presence of serum, that is serum *per se* did not suppress T7 efficacy in generating Mango II aptamer. Figure 14 shows that in the presence of equine serum or fetal calf serum (FCS) T7 RNA polymerase continued to generate Mango II.

There was no evidence that serum inadvertently suppressed T7 activity. Next, it was tested whether in the presence of serum, AR remained responsive to testosterone as the example ligand. Reactions were established as described above, except this time water component was replaced with serum into which testosterone (or ethanol as vehicle) had been spiked. Figure 15 shows that the reaction is not compromised if serum is present as a reaction component. This result is very important as shows the assay is capable of detecting androgen levels in a biologically relevant sample for clinical or sports doping application, for example.

In the next phase of testing, the Androgen Assay Prototype 2 was tested for its ability to detect endogenous androgens deconjugated and extracted from equine urine samples. Racehorse urine samples were collected on race day and steroids deconjugated and extracted using routine processes. The extracted steroids were resuspended in ethanol and subjected to the assay.

Figure 16 shows that the Androgen Assay Prototype 2 was able to detect high levels of androgens in the urine samples from colts (male horses) while not being able to detect high levels from the geldings (castrated male horses). Testosterone and spiked trenbolone (AAS) in gelding urine were used as controls.

Data from Figure 15 and Figure 16 show that the assay is able to detect androgenic molecules in biological matrices including serum and urine.

Although the invention has been described by way of example, it should be appreciated that variations and modifications may be made without departing from the scope of the invention as defined in the claims. Furthermore, where known equivalents exist to specific features, such equivalents are incorporated as if specifically referred in this specification.

All patents, publications, scientific articles, web sites, and other documents and materials referenced or mentioned herein are indicative of the levels of skill of those skilled in the art to which the invention pertains, and each such referenced document and material is hereby incorporated by reference to the same extent as if it had been incorporated by reference in its entirety individually or set forth herein in its entirety. Applicants reserve the right to physically incorporate into this specification any and all materials and information from any such patents, publications, scientific articles, web sites, electronically available information, and other referenced materials or documents.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed. Thus, it will be understood that although the present invention has been specifically disclosed by preferred embodiments and optional features, modification and variation of the concepts disclosed herein may be resorted to by those skilled in the art, and that such modifications and variations are considered to be within the scope of this invention as described herein, and as defined by the appended claims.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other examples are within the following claims. In addition, where features or aspects of the invention are described in terms of Markush groups, those skilled in the art will recognize that the invention is also thereby described in terms of any individual member or subgroup of members of the Markush group.

### ASPECTS OF THE INVENTION

1. A test kit for screening a test sample for the presence of a ligand capable of eliciting a steroid hormone genomic response, the test kit comprising:
   (i) a steroid hormone receptor that is capable of forming a ligand-receptor complex with a ligand from the test sample; and
   (ii) a nucleic acid molecule comprising:
      (a) a polymerase promoter sequence;
      (b) a response element that is capable of being bound by the ligand-receptor complex; and
      (c) a reporter construct
      where the response element (b) is located between the promoter sequence (a) and the reporter construct (c), and (a), (b) and (c) are operably linked; and
   (iii) a single polypeptide polymerase; and
   wherein, the presence of a ligand in the test sample is detected by measuring a reduction or inhibition in transcription of the reporter construct caused by binding of the ligand-receptor complex to the response element when the sample is combined with the test kit.
2. The test kit according to aspect 1, further comprising a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.
3. The test kit according to aspect 2, wherein the relative amount of HSP90 to steroid hormone receptor is x:1, where x is the amount of HSP90 and is defined as [1.0 ≤ x ≤ 5.0].
4. The test kit according to any one of aspects 1 to 3, wherein the relative amount of steroid hormone receptor to nucleic acid molecule is y:1, where y is the amount of steroid hormone receptor and is defined as [7.0 ≤ y ≤ 10.0].
5. The test kit according to any one of aspects 1 to 4, wherein the polymerase is T7 RNA polymerase.
6. The test kit according to aspect 5, wherein the polymerase promoter sequence is defined by SEQ ID NO: 1.
7. The test kit according to any one of aspects 1 to 6, wherein the reporter construct comprises a sequence encoding an RNA aptamer capable of binding to a fluorophore.
8. The test kit according to aspect 6 or aspect 7, wherein the RNA aptamer is Mango II, and optionally comprises the F30 scaffold.
9. The test kit according to any one of aspects 1 to 8, further comprising nucleoside triphosphates.
10. The test kit according to any one of aspects 1 to 9, wherein the steroid hormone receptor is selected from the group consisting of androgen receptor (AR); estrogen receptor alpha (ER-α) and estrogen receptor beta (ER-β); progesterone receptor A (PRA) and progesterone receptor B (PRB); mineralocorticoid receptor (MR); and glucocorticoid receptor (GR).
11. The test kit according to any one of aspects 1 to 10, wherein the response element is selected from:
   a. an androgen response element (ARE) including, but not limited to, a sequence comprising 5'-AGAACAnnnTGTTCT-3' (SEQ ID NO: 4), wherein n is A, T, G or C;
   b. an estrogen response element (ERE) including, but not limited to, a sequence comprising 5'-AGGTCAnnnTGACCT-3' (SEQ ID NO: 8), wherein n is A, T, G or C;
   c. a progesterone response element (PRE) including, but not limited to, a sequence comprising 5'-GGTACAAACTGTTCT-3' (SEQ ID NO: 10;
   d. a mineralocorticoid response element (MRE) including, but not limited to, a sequence comprising 5'-AGAACAnAATGTTCT-3' (SEQ ID NO: 12), wherein n is A, T, G or C; and
   e. a glucocorticoid response element (GRE) including, but not limited to, a sequence comprising 5'-AGAACAnAATGTTCT-3' (SEQ ID NO: 12), wherein n is A, T, G or C.
12. The test kit according to any one of aspects 1 to 11, wherein the test kit is configured to detect a ligand that binds to an androgen receptor, and the nucleic acid molecule comprises a sequence defined by SEQ ID NO: 14, or wherein the test kit is configured to detect a ligand that binds to an estrogen receptor, and the nucleic acid molecule comprises a sequence defined by SEQ ID NO: 15.
13. An assay method for detecting a ligand in a sample which ligand is capable of eliciting a steroid hormone genomic response, the assay method comprising the steps of:
   (i) contacting a sample with:
      (a) a steroid hormone receptor that forms a ligand-receptor complex with a ligand from the sample; and
      (b) a nucleic acid molecule comprising:
         (1) a polymerase promoter sequence;
         (2) a response element that is bound by the ligand-receptor complex; and
         (3) a reporter construct
         where the response element (b) is located between the promoter sequence (a) and the reporter construct (c), and (a), (b) and (c) are operably linked;
      (c) a single polypeptide polymerase; and
      (d) nucleoside triphosphates; and
   (ii) measuring a reduction or inhibition in transcription of the reporter construct caused by binding of the ligand-receptor complex to the response element,
   wherein, a measured reduction or inhibition in transcription of the reporter construct reflects detection of a ligand in the sample.
14. A method for determining the doping status of an athlete, the method comprising performing a test kit or assay method according to any one of aspects 1 to 13 on a sample obtained from the athlete to ascertain if the sample comprises a ligand sufficient to bind to and activate a steroid hormone receptor and cause a change in a physical property of the reporter construct, wherein a change in a physical property of the reporter construct provides information about the doping status of the athlete.
15. A method according to aspect 14, wherein the athlete is selected from a human athlete, an equine athlete, a canine athlete and a camelid athlete.

## Claims

1. A cell-free test kit for screening a test sample for the presence of a ligand capable of eliciting a steroid hormone genomic response, the test kit comprising:
(i) a steroid hormone receptor that is capable of forming a ligand-receptor complex with a ligand from the test sample; and
(ii) a nucleic acid molecule comprising:
(a) a single polypeptide polymerase promoter sequence;
(b) a response element that is capable of being bound by the ligand-receptor complex; and
(c) a reporter construct
where the response element (b) is located between the promoter sequence (a) and the reporter construct (c), and (a), (b) and (c) are operably linked; and
(iii) a polymerase consisting of a single polypeptide.

2. The test kit according to claim 1, further comprising a steroid hormone receptor cofactor selected from heat shock protein 90 (HSP90), a complex of HSP90 and heat shock protein 70 (HSP70), a complex of HSP90, HSP70 and heat shock protein 40 (HSP40), a complex of HSP90, HSP70, HSP40 and p23, a complex of HSP90, HSP70, HSP40, p23 and heat shock protein organizing protein (Hop), a complex of HSP90, HSP70, HSP40, p23, Hop and 48kD Hip protein (Hip), a complex of HSP90, HSP70, HSP40, p23, Hop, Hip and p60, and a complex of HSP90, HSP70, HSP40, p23, Hop, Hip, p60 and FKBP52.

3. The test kit according to claim 2, wherein the relative amount of HSP90 to steroid hormone receptor is x:1, where x is the amount of HSP90 and is defined as [1.0 ≤ x ≤ 5.0].

4. The test kit according to any one of claims 1 to 3, wherein the relative amount of steroid hormone receptor to nucleic acid molecule is y: 1, where y is the amount of steroid hormone receptor and is defined as [7.0 ≤ y ≤ 10.0].

5. The test kit according to any one of claims 1 to 4, wherein the polymerase is T7 RNA polymerase.

6. The test kit according to any one of claims 1 to 5, wherein the polymerase promoter sequence is defined by SEQ ID NO: 1.

7. The test kit according to any one of claims 1 to 6, wherein the reporter construct comprises a sequence encoding an RNA aptamer capable of binding to a fluorophore.

8. The test kit according to claim 7, wherein the RNA aptamer is selected from Mango I, Mango II, Mango III and Mango IV, Spinach, iSpinach, Baby Spinach, Broccoli and Malachite Green.

9. The test kit according to claim 7 or claim 8, wherein the RNA aptamer is Mango II, and comprises the F30 scaffold.

10. The test kit according to any one of claims 1 to 9, wherein the steroid hormone receptor is selected from the group consisting of androgen receptor (AR); estrogen receptor alpha (ER-α) and estrogen receptor beta (ER-β); progesterone receptor A (PRA) and progesterone receptor B (PRB); mineralocorticoid receptor (MR); and glucocorticoid receptor (GR).

11. The test kit according to any one of claims 1 to 10, wherein the response element is selected from:
a. an androgen response element (ARE) comprising a sequence 5'-AGAACAnnnTGTTCT-3' (SEQ ID NO: 4), wherein n is A, T, G or C;
b. an estrogen response element (ERE) comprising a sequence comprising 5'-AGGTCAnnnTGACCT-3' (SEQ ID NO: 8), wherein n is A, T, G or C;
c. a progesterone response element (PRE) comprising a sequence comprising 5'-GGTACAAACTGTTCT-3' (SEQ ID NO: 10;
d. a mineralocorticoid response element (MRE) comprising a sequence comprising 5'-AGAACAnAATGTTCT-3' (SEQ ID NO: 12), wherein n is A, T, G or C; and
e. a glucocorticoid response element (GRE) comprising a sequence comprising 5'-AGAACAnAATGTTCT-3' (SEQ ID NO: 12), wherein n is A, T, G or C.

12. The test kit according to any one of claims 1 to 11, wherein the nucleic acid molecule comprises a sequence set forth in any one of SEQ ID Nos: 14 to 73.

13. The test kit according to any one of claims 1 to 12, wherein the test kit is configured to detect a ligand that binds to an androgen receptor, and the nucleic acid molecule comprises a sequence defined by SEQ ID NO: 14, or wherein the test kit is configured to detect a ligand that binds to an estrogen receptor, and the nucleic acid molecule comprises a sequence defined by SEQ ID NO: 15.

14. The test kit according to any one of claims 1 to 13, further comprising nucleoside triphosphates.
